# EUROPÄISCHE PATENTSCHRIFT

(11) **EP 1 487 346 B1**
(45) Veröffentlichungstag und Bekanntmachung des Hinweises auf die Patenterteilung: **31.08.2005**
(21) Anmeldenummer: 03720187.8
(22) Anmeldetag: 17.03.2003
(51) Int. Cl.: A61B 10/00

(54) **VAKUUM-BIOPSIEVORRICHTUNG**
VACUUM BIOPSY DEVICE
DISPOSITIF POUR BIOPSIE SOUS VIDE

(30) Priorität: 19.03.2002 DE 20204363 U; 19.03.2002 DE 20204362 U; 19.03.2002 DE 20204361 U; 19.03.2002 DE 10212156; 19.03.2002 DE 10212139; 19.03.2002 DE 10212155; 19.06.2002 DE 20209525 U; 19.06.2002 DE 20209530 U; 02.08.2002 DE 20211934 U; 17.10.2002 DE 20215962 U
(43) Veröffentlichungstag der Anmeldung: 22.12.2004
(73) Patentinhaber: Bard Dublin ITC Limited, West Sussex RH11 9BP (GB)
(72) Erfinder: HESKE, Norbert, 82288 Kottgeisering (DE); HESKE, Thomas, 82284 Grafrath (DE)
(74) Vertreter: Tomlinson, Edward James
(86) Internationale Anmeldenummer: PCT/DE2003/000844
(87) Internationale Veröffentlichungsnummer: WO 2003/077767

(56) Entgegenhaltungen:
- US-A- 5 976 164
- US-A1- 2001 014 779

## Beschreibung

### Technisches Gebiet

Die Erfindung bezieht sich auf eine Biopsievorrichtung zur Entnahme von Gewebeproben, die aus einem Handstück, besteht, in das eine hohle Biopsienadel eingelegt wird, wobei ein Teil des über das Handstück hinausragenden Teils der Biopsienadel mit seinem Probeentnahmeraum in das zu untersuchende Gewebe eingebracht wird und das Gewebe mittels Vakuum in den Probeentnahmeraum eingesaugt und anschließend mittels einer Probeabtrenneinrichtung abgetrennt und anschließend entnommen wird.

### Stand der Technik

Aus der GB 2018601A ist bereits ein Verfahren und eine Vorrichtung zum Herausschneiden von Gewebe bekannt, bei der unter Vakuumeinfluss das Gewebe in der Biopsienadel in einen Schneidebereich eingesaugt wird. Um in der Hohlnadel ein Vakuum zu erzeugen ist das Handstück, in das die Hohlnadel integriert ist, über Leitungen mit einem außerhalb des Handstücks angeordneten Vakuumerzeuger verbunden. Das Abschneiden der Probe erfolgt über eine Schneideinrichtung, die in der Hohlnadel längsbeweglich angeordnet ist. Die herausgetrennte Probe wird in der Nadel gelagert. Nach dem Herausziehen der Nadel aus dem Gewebe wird die herausgeschnittene Probe aus der Nadelspitze herausgespült; deshalb ist das Handstück über weitere Leitungen mit außerhalb des Handstücks liegenden Geräten verbunden. Das in der Hohlnadel angelegte Vakuum wird über in die Leitungen integrierte Steuerelemente geregelt.

Aus der EP 0890 339 A1 ist eine weitere Biopsieeinrichtung bekannt, bei der unter Vakuumeinfluss die Probe entnommen wird. In dem Handstück, in das die Biopsienadel mit Schneideinrichtung integriert ist und eingelegt wird, ist die Biopsienadel über Schlauchverbindungen und Leitungen mit einem externen Vakuumerzeuger sowie Steuergeräten verbunden. Das Vakuum wird über einen an die Außenhülse der Biopsienadel angeformten Kanal an den Probeentnahmeraum von unten herangeführt.

Die Abtrennvorrichtung ist im Hohlraum der Biopsienadel längsverschieblich angeordnet. Durch eine rotierende Bewegung, kombiniert mit einem manuellen Längsschub schneidet die Abtrennvorrichtung die Probe aus dem Gewebe. Die Probe wird im Hohlkanal der Abtrennvorrichtung transportiert. Eine ähnliche Anordnung zeigt auch die US-PS 5526 822, wobei hier insbesondere verschiedene Vakuumzuführungen an den Probeentnahmeraum, wie die Anordnung für Schneideinrichtungen, in der Hohlnadel oder koaxial als Schneidhülse, außen bekannt sind .
Bei beiden Biopsieeinrichtungen, die eine Probeentnahme unter Vakuum zulassen, ist das Handstück der Biopsievorrichtung über mindestens einen Verbindungsschlauch und/oder Versorgungskabel mit einer oder mehreren externen Versorgungseinheiten in ihrer Bewegungsfreiheit eingeschränkt; darüber hinaus sind die Einrichtungen für das Erzeugen von Vakuum aufwendig, insbesondere hinsichtlich der Regeleinrichtungen. Die Probe wird durch in dem Nadelhohlraüm längsbewegliche, rotierende Abtrennvorrichtungen herausgeschnitten.

Aus der DE 40 41 614 C1 ist weiterhin ein Sogbiopsiegerät bekannt, das als Handgerät ausgebildet ist und über eine Unterdruckquelle sowie einen Biopsiekanülenanschluss verfügt, der über einen außerhalb des Handstücks liegende biegsame Welle in Rotation versetzbar ist. An den Biopsiekanülenanschluss ist eine als Hohlkanüle ausgebildete Biopsiekanüle ansetzbar, die vorzugsweise über eine distalseits angeschärfte, umlaufende Schneidkante verfügt, längs deren Hohlkanal ein mittels der Unterdruckquelle, die als Kolben-Zylindereinheit ausgebildet ist, Unterdruck applizierbar ist, sobald die Hohlkanüle an eine bestimmte intrakorporale Gewebestelle positioniert worden ist.

Ein ähnlich unterdruckunterstützendes Biopsiegerät ist der WO 96/28097 zu entnehmen, das zwar keine in Rotation versetzbare Hohlkanüle vorsieht, doch über eine innerhalb eines Handgerätes angeordnete Spritzenkolbenanordnung zur Unterdruckerzeugung verfügt.

Die DE 100 34 297 A1 beschreibt im Unterschied zu der vorstehenden Sogbiopsieanordnung mit nur einer einzigen Hohlnadel ein Gewebeentnahme-Endoskopieinstrument, das eine Biopsienadelanordnung aufweist, die eine an ihrem distalen Ende umlaufend angeschärfte Hohlnadel und eine innerhalb der Hohlnadel geführte hohle Biopsienadel vorsieht, wobei die innengeführte Biopsienadel an ihrem distalen Ende eine Ausnehmung zur Gewebeprobeentnahme aufweist. Proximalseitig zur hohlen Biopsienadel ist ein Ansauginstrument zur Unterdurckerzeugung vorgesehen. Eine Gewebeentnahme erfolgt derart, dass die Biopsienadelanordnung in einer gemeinsamen Stellung in einen zu untersuchenden Gewebebereich vorgeschoben wird, wobei die Biopsienadel eine distale Spitze vorsieht, die distalseits aus der Hohlnadel ein Stück emporragt, um einerseits den Eindringvorgang der Biopsienadelanordnung in das Gewebe zu begünstigen und andererseits das Eindringen von Gewebe in das Innere der Hohlnadel zu verhindern. Bei geeigneter Positionierung der Biopsienadelanordnung innerhalb des Gewebes wird die Hohlnadel ein definiertes Stück proximalwärts gezogen, wobei die innenliegende Biopsiekanüle in ihrer Position verbleibt und die Ausnehmung freigegeben wird. Der längs der Biopsienadel angelegte Unterdruck bewirkt ein aktives Absenken bzw. Einziehen von umliegenden Gewebeteilen in die Ausnehmung, Durch kontrolliertes distalseitiges Vorschieben der Hohlnadel mit ihrem angeschärften distalen Ende über die Biopsienadel wird ein Teil des Gewebes abgetrennt und innerhalb der Ausnehmung der Biopsienadel eingeschlossen. Durch gemeinsames Zurückziehen der Biopsienadelanordnung wird sodann die abgetrennte Gewebeprobe exkorporal für Untersuchungszwecke entnommen. Der gesamte vorbeschriebene Gewebeentnahmevorgang erfolgt derart, dass die Nadelbewegungen sowie die Unterdruckapplizierung einzeln und getrennt voneinander manuell vorgenommen werden.

Demgegenüber ermöglicht die Biopsienadelanordnung, die in der WO 98/25522 beschrieben ist, eine Federkraft beaufschlagte Relativbewegung zwischen innenliegender hohler Biopsienadel und der die Biopsienadel umgebenden äußeren Hohlnadel. Auch in diesem Fall wird die Biopsienadel zur Gewebeaufnahme distalseits zur angeschärften distalen Spitze der Hohlnadel positioniert, wobei eine Unterdruckquelle zur gezielten Unterdruckversorgung durch die hohle Biopsienadel hindurch in den Bereich ihrer Ausnehmung vorgesehen ist und den Gewebeeinbringvorgang unterstützt. Der Positioniervorgang der Biopsienadel relativ und letztlich innerhalb des zu untersuchenden Gewebebereiches erfolgt ausschließlich manuell. Ein derartige Positionierung führt insbesondere bei der Untersuchung harter Gewebebereiche nur zu unbefriedigenden Biopsieergebnissen.

In der US 2001/0011156 A1 ist ebenfalls eine vakuumunterstützte Biopsievorrichtung beschrieben, die ein kompakt gestaltetes Handgerät vorsieht, in dessen Gehäuse alle für den Nadelantrieb der Biopsienadelanordnung notwendigen Antriebselemente vorgesehen sind. Eine Unterdruckquelle ist jedoch getrennt zum Handgerät vorgesehen, die über eine entsprechende Versorgungsleitung mit der Nadelanordnung innerhalb des Handgerätes an einer geeigneten Verbindungsstelle anschließbar ist.

### Darstellung der Erfindung

Ausgehend von dem als nächstkommenden Stand der Technik angesehenen DE 100 34 297 A1 liegt der Vakuum-Biopsievorrichtung zur Entnahme von Gewebe die Aufgabe zugrunde das Handstück so zu gestalten, dass der Ablauf der Probeentnahme nach dem Einsetzen mittels einer oder ohne eine Koaxialkanüle mit einer Hand bedient werden kann. Die Vorteile der bekannten High-Speed-Biopsiepistole ohne Vakuum, nämlich das schnelle Eindringen der den Probeentnahmeraum tragenden Nadeleinheit in das zu entnehmende Gewebe, soll jedochweiterhin erhalten bleiben. Weiterhin soll die Vakuum-Erzeugungsvorrichtung einfach, zuverlässig und unkompliziert aufgebaut sein. Die Probeentnahme soll so erfolgen, dass dem Pathologen eine ausreichende, nicht verdrillte Gewebemenge zur Beurteilung vorliegt.

Die erfindungsgmäße Lösung besteht deshalb darin, dass der Spannschlitten mittels elektrischer Motorkraft gegen die Wirkung einer Feder in Spannstellung gebracht wird, auf dem im Handstück gelagerten Spannschlitten die Nadeleinheit angeordnet ist und der Probeentnahmeraum in das Gewebe nach Entriegelung des vorgespannten Spannschlittens eingeschossen wird, die Vakuum-/Druckerzeugungsvorrichtung (5) sowie weitere Steuerungs- und Versorgungsvorrichtungen in das Gehäuse des Handstücks (1) integriert sind, und das Verbindungselement (4) von der Biopsienadel (3) zur Vakuum-/Druckerzeugungseinheit (5) unmittelbar am Gehäuse angeordnet ist, die Vakuum-/Druckerzeugungsvorrichtung (5) aus einer steuerbaren Kolben-/Zylindereinheit (69) besteht, die eine Belüftungsöffnung (67) aufweist, so dass in der Vakuum-/Druckerzeugungsvorrichtung zum Auswurf der Probe Überdruck erzeugt werden kann, dass alle Antriebe elektrisch betätigt sind und dass der Antrieb für den Spannschlitten auch als Antrieb für die Schneidhülse verwendet wird, dass die Biopsiehohlnadel von einer außen liegenden, koaxialen Schneidhülse umgeben ist und dass auf der Frontseite des Gehäuses eine Platine für die Betätigung der Elektronik angeordnet ist, in die der Entraster des Spannschlittens integriert ist.
Durch die Anordnung aller benötigten Vorrichtungen im Handstück ist das Handstück frei beweglich; darüber hinaus werden ausschließlich elektrische hochtourige Antriebe verwendet; mit dem gleichen Antrieb wird der Spannschlitten und die Probeabtrennvorrichtung angetrieben. Dadurch entsteht ein kompaktes und von anderen Versorgungseinheiten unabhängiges Gerät. Die Antriebe können in einem relativ kleinen Gehäuse untergebracht werden. Selbst die Elektronik und die Bedien- und Kontrollinstrumente sind an dem Gehäuse angeordnet, bzw. in ihm untergebracht. Dies trifft auch für die Stromversorgung und die Verbindungselemente zu. Dadurch ist es möglich, Teilprozesse zu einem Steuerungsschritt zusammenzufassen und die Bedienung zu vereinfachen, so dass die Bedienung einhändig durchgeführt werden kann.

Um die Vakuumerzeugungsvorrichtung besonders einfach und zuverlässig zu gestalten, hat sich für das Erzeugen des Vakuums und des Überdrucks der Einsatz einer Kolben-/Zylindereinheit mit Belüftungsmöglichkeit bewährt. Besonders vorteilhaft ist hierbei die Verwendung einer bekannten Spritzen-/Kolbeneinheit, mit einer zusätzlich im oberen Teil des Spritzenkörpers angeordneten Belüftungöffnung, die zum Abbau des Vakuums durch weiteres Zurückziehen des Spritzenkolbens geöffnet wird. Durch Steuerung des Spindelantriebs der Kolbenspindel kann die gleiche Kolben/Spritzeneinheit, je nach Erfordernis, von der Erzeugung eines Vakuums auf die Erzeugung eines Überdrucks umgesteuert werden, wobei zum Abbau des Vakuums eine im oberen Teil angeordnete Belüftungsöffnung dient, durch die Luft einströmt, die im nachfolgenden Schritt komprimiert wird.
Um die Bewegung des Kolbens, insbesondere im Hinblick auf die Umsteuerung vom Aufbau eines Vakuums, auf Abbau eines Vakuums und Erzeugung eines Überdrucks steuern zu können, hat sich als Antrieb ein Spindelantrieb mit elektrischem Gleichstrommotor mit nachgeschaltetem Untersetzungsgetriebe als vorteilhaft erwiesen.

Die gemessene Umdrehungszahl des Motors stellt unmittelbar ein Maß für die Längsverschiebung des Kolbens dar. Da es sich um einen Gleichstrommotor mit hoher Drehzahl handelt, dessen Abtriebsdrehzahl über ein Untersetzungsgetriebe erheblich reduziert wird, ist die Längsbewegung der Spindel genau steuerbar. Über entsprechende Soll-Wertvorgaben in der Steuerelektronik , z.B. die Umdrehungszahl des Motors, kann die Länge des Spindelweges und damit die Größe des Vakuums und der Überdruck vorgegeben werden.

Da bei jedem Patienten eine sterile Biopsienadel verwendet wird, hat sich eine Trennung von sterilen Teilen und sonstigen, lediglich desinfizierten Teilen, die fest mit dem Handstück verbunden sind, als vorteilhaft erwiesen. Aus diesem Grunde ist es günstig, die Vakuum-/Druckerzeugungsvorrichtung, die Biopsienadel mit Schneidhülse und der mit Biopsienadel und Schneidhülse verbundenen Teile wie Biopsienadelträger, Antriebselemente und Kunststoffteil einschließlich Verbindungselement und Führungsrolle als selbständiges, leicht einlegbares und herausnehmbares steriles Einlegeelement, auszubilden. Der Raum für das Einlegeelement ist aus Gründen der Reinigung des Handstücks von den sonstigen Antriebselementen durch Abdeckungen getrennt.
Das flexible Verbindungselement wird einfachheitshalber als flexibler Schlauch ausgebildet, so dass es sich den Verschiebungswegen des Spannschlittens anpassen kann. Um den Schlauch gegenüber der Biopsienadel am proximalen Ende verdrehen zu können, ist ein zusätzliches, drehgelagertes Kunststoffteil im fest mit der Biopsienadel verbundenen Kunststoffteil angeordnet, an dem der Schlauch befestigt ist.
Um dem mit der Spindelhülse verbundenen Zahnrad zum Antrieb der Spindelhülse eine Längsbewegung zu ermöglichen, z.B. beim Auslösen des Spannschlittens, ist eine Zahnwalze als Antrieb vorgesehen.
Um über den Biopsienadelträger ein Spannen des Spannschlittens durch Verdrehen der Schneidhülse herbeizuführen, wird beim Spannen das stirnseitige Zahnrad an der Gewindespindelhülse an einer Halterung des Basisblocks abgestützt, so dass der Biopsienadelträger nach rechts bewegt wird, während die Schneidhülse ihre Position beibehält.
Die Verrastung des Spannschlittens weist einen doppelarmigen Hebel auf, dessen einer Arm unter Federdruck in die Ausnehmung des Spannschlittens eingreift. Um die Spannvorrichtung für verschiedene Biopsienadeln mit verschiedenen Einschusstiefen, z.B. von 15 - 25 mm verwenden zu können, bedarf es z.B. lediglich einer Anpassung der einrastenden Hebellänge und einer entsprechenden Vorgabe in der Elektronik Das an die Biopsienadel angefügte Kunststoffteil ermöglicht mittels einer Rändelscheibe ein Verdrehen des Probeentnahmeraums. Durch das Zusammenwirken des Vielkants des Kunststoffteils mit dem Biopsienadelträger ist die Biopsienadel in der gewünschten Stellung arretierbar. Eine auf der Rändelscheibe angebrachte Kerbe zeigt dem Bediener an, in welcher radialen Position sich die Öffnung des Probeentnahmeraums befindet.

Der Querschnitt der Biopsiehohlnadel wird zum Probeentnahmeraum hin durch eine Einengung, einen Stopfen oder eine Lippe, begrenzt. Diese Einengung beträgt ca. 60 - 75% der Höhe und deckt den oberen, offenen Teil des Probeentnahmeraums von oben her ab. Diese Verengung vor dem Probeentnahmraum bewirkt, dass das Vakuum vom Boden her - beim Öffnen des Probeentnahmeraums - (also beim Zurückfahren der Schneidhülse) das zu untersuchende Gewebe einsaugt. Die Verengung verhindert zusätzlich ein Eindringen von Gewebe in den hinteren Teil des Nadelhohlraums. Beim Auswurf der Probe bewirkt die Verengung eine Druckerhöhung im Probeentnahmeraum, was den Reinigungseffekt insbesondere im Probeentnahmeraum verbessert. Durch das Anlegen des Vakuums wird das Gewebe der Probe in den Innenraum des Probeentnahmeraums eingesaugt und haftet gewissermaßen an der Innenwand. Zur besseren Haftung können im Innenraum des Probeentnahmeraums zusätzliche Mittel vorgesehen sein. Da die Schneidhülse auf dem Außendurchmesser der Biopsienadel angeordnet ist und somit die Abtrennung des Gewebes außenseitig erfolgt, wird das am Innenraum anhaftende Gewebe durch die außenseitige Anordnung der Schneidhülse nicht durch die Schneideinrichtung von der Innenwand abgelöst. Darüberhinaus kann das Gewebe nicht in den Hohlraum der rotierenden Schneideinrichtung eindringen und darin anhaften. Die Führung der querschnittsrunden Schneidhülse auf der Außenseite der querschnittsrunden Biopsienadel hat durch diese Anordnung den Vorteil, dass kein Verdrillen (Verdrehen) der Probe durch die Schnittdrehung der Schneideinrichtung stattfindet und somit eine wesentliche Voraussetzung für die Beurteilung des Gewebes durch den Pathologen erfüllt wird. Um ein gutes Anhaften der Probe im Innenraum zu erhalten ohne den Füllungsgrad zu beeinträchtigen, ist der Probeentnahmeraum so gestaltet, dass ca. 25% des Probeentnahmeraumquerschnitts für das Einsaugen der Probe geöffnet ist, d.h. der größere Teil des Umfangs ist geschlossen.

Die außenseitige Anordnung der koaxialen Schneidhülse bewirkt zusätzlich, dass eine größere Probe entnommen werden kann, als bei innenseitiger Anordnung der Schneidhülse.
Da die Probe mit Unterstützung eines aufgebauten Drucks aus dem Probeentnahmeraum ausgeworfen wird, findet beim Herausnehmen des Gewebes keine Schädigung des Gewebes statt.
Durch die zentrale Anordnung des Basisblocks im Zentrum des Gehäuseinnenraums wird das Gehäuse selbst von Querkräften, die durch die Antriebselemente entstehen, entlastet. Darüber hinaus ist der Austausch von Antrieben, wie auch des Spannschlittens leicht durchführbar, da hierfür nur die Verbindungen zum Gehäuse gelöst werden müssen. Vorteilhaft ist auch, dass die durch den Kunststoffspannschlitten erzeugten Stöße durch den Basisblock absorbiert werden.

Die Lagerung der Biopsienadel/Schneidhülse in einem Biopsienadelträger aus Kunststoff hat u.a. den Vorteil, dass durch die angeformten Gleitflächen ein störungsfreies Gleiten auf den Gegenflächen des Basisblocks und des angeformten Blocks ermöglicht wird. Der Biopsienadelträger überträgt die Kräfte vom Spindelantrieb der Schneidhülse auf den Spannschlitten. Da sich der Spindelantrieb bei der Verstellung des Spannschlittens auf der Halterung des Basisblocks abstützt und beim Verdrehen der Spannhülse frei gleiten kann (das Zahnrad kann in der Zahnwalze axial gleiten), kann der Antrieb für beide Bewegungsvorgänge (Spannen des Spannschlittens, Öffnen und Schließen des Probeentnahmeraums mittels der Schneidhülse) genutzt werden. Der im Gehäuseendteil integrierte Mikroschalter, der durch das Schließen des Gehäusedeckels bei eingelegter Vakuum/Druckerzeugungsvorrichtung die Stromzufuhr ab-, bzw. und anschaltet, wie der an dem Biopsienadelträger angebrachte Sicherungsflügel sind Sicherheitseinrichtungen, die verhindern, dass bei geöffnetem Gehäusedeckel ein Spannen des Spannschlittens erfolgt. Außerdem soll ein Öffnen des Gehäusedeckels mit gespannter Nadel ausgeschaltet werden.

Die im Gehäuseenddeckel gelagerte und von der Biopsienadel mit Schneidhülse durchdrungene Führungsrolle wirkt mit der vorher in das Gewebe gesetzten Kanüle zusammen. Da auf das proximale Ende der vorher gesetzten Koaxialkanüle ein Dichtelement aufgesetzt wird, das mit der Schneidhülse zusammenwirkt, wird verhindert, dass Luft zwischen der Kanüle und der Schneidhülse eindringt. Die auf die Kanüle aufgesetzte Führungsrolle verhindert, dass eine Verschmutzung des Innengehäuses stattfindet und dass das nicht sterile Handstück die Koaxialkanüle berührt. Durch die am Handstück angeordnete Platine mit integrierten Leuchtdioden und Schaltern sowie Pictogrammen wird eine einfache Bedienerführung erzielt.
Die Abdeckplatte kann auch als Träger für Mikroschalter oder Fotozellen verwendet werden.
Die Einlegehilfe ermöglicht das leichtere Einlegen des sterilisierten Austauschelements.

Um sicherzustellen, dass beim Verschließen des Probeentnahmeraums das Gewebe sicher durchtrennt wird, wird die Schneidhülse über das distale Ende des Probeentnahmeraums um ca. 2 mm hinaus zur Nadelspitze gefahren.
Zur Vermeidung von Fehlbedienung sind die Vorgänge "Spannen des Spannschlittens" und "Auswurf der Probe" mit Verzögerungsschaltungen versehen, Zur Erhöhung der Sicherheit kann es zweckmäßig sein, für die Leuchtdioden bei Vorgängen, die im Gewebe abgewickelt werden, z.B. "Probe heraustrennen", eine andere Farbe zu wählen, als bei den Vorgängen, die außerhalb des Gewebes ablaufen, z.B. "Probe auswerfen".

Beim Einsatz einer Koaxialkanüle, in die die Nadeleinheit eingesetzt wird, um beispielsweise eine genaue Positionierung zu erhalten, ist darauf zu achten, dass zwischen Außenumfang Nadel und Innenseite der Koaxialkanüle keine Luft beim Aufbau eines Vakuums eindringen kann. Deshalb ist an der proximalen Seite des Koaxialkanülenrohres ein Dichtelement vorgesehen.

Da die Einschusstiefe der Nadeleinheit durch den Spannweg des Spannschlittens vorgegeben ist, es sei denn, man sieht Mittel im Handstück für verschiedene Einschusstiefen vor, hat sich der Einsatz von Distanzstücken zwischen Koaxialkanüle und Führungsrolle als besonders vorteilhaft erwiesen. Das Distanzstück wird auf die Nadeleinheit aufgefädelt und sitzt distalseits auf dem proximalen Ende der Koaxialkanüle, und proximalseits auf einer im Handstück angeordneten Führungsrolle auf. Dadurch wird bei gleichem, geräteseits bedingtem Einschussweg die Eindringtiefe um die Länge des Distanzstücks verringert; dies führt zu erleichterten Produktionsbedingungen.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben.

Es zeigt:
- Fig. 1: Biopsievorichtung mit geöffnetem Gehäusedeckel (perspektivisch)
- Fig. 2: Gehäusefeste Einheit der Biopsievorrichtung (ohne Gehäuseboden und Deckel) und austauschbare Biopsieeinheit ;(perspektivisch) getrennt dargestellt
- Fig. 3: Längsschnitt A - A durch die Biopsienadel in Fig.1
- Fig. 4: Querschnitt B - B in Fig. 1 (linker Gehäuseteil)
- Fig. 5: Querschnitt C - C in Fig. 1 (rechter Gehäuseteil)
- Fig. 6: rechter Gehäuseenddeckel (Innenseite) mit integriertem Mikroschalter

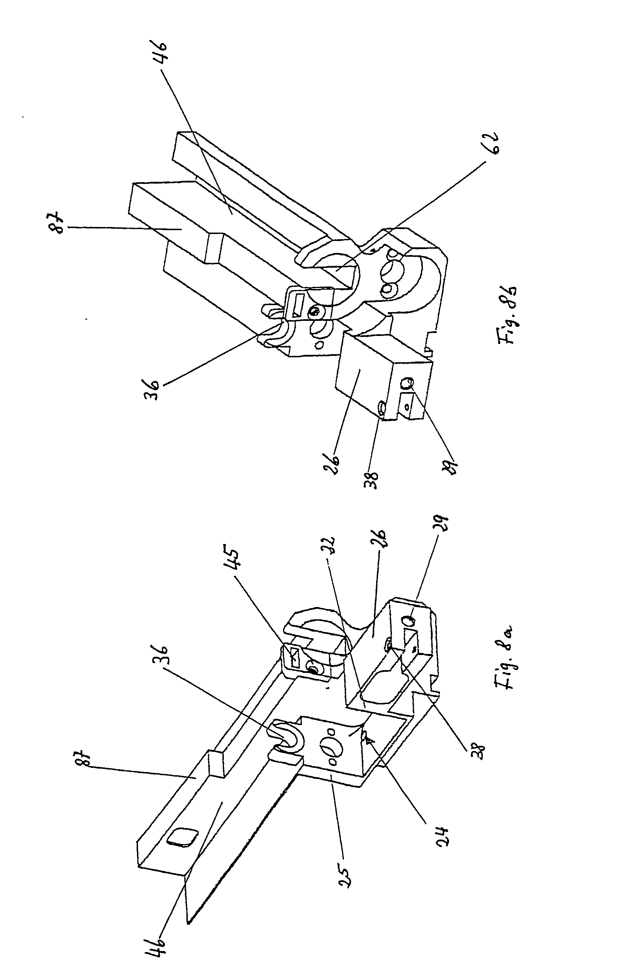

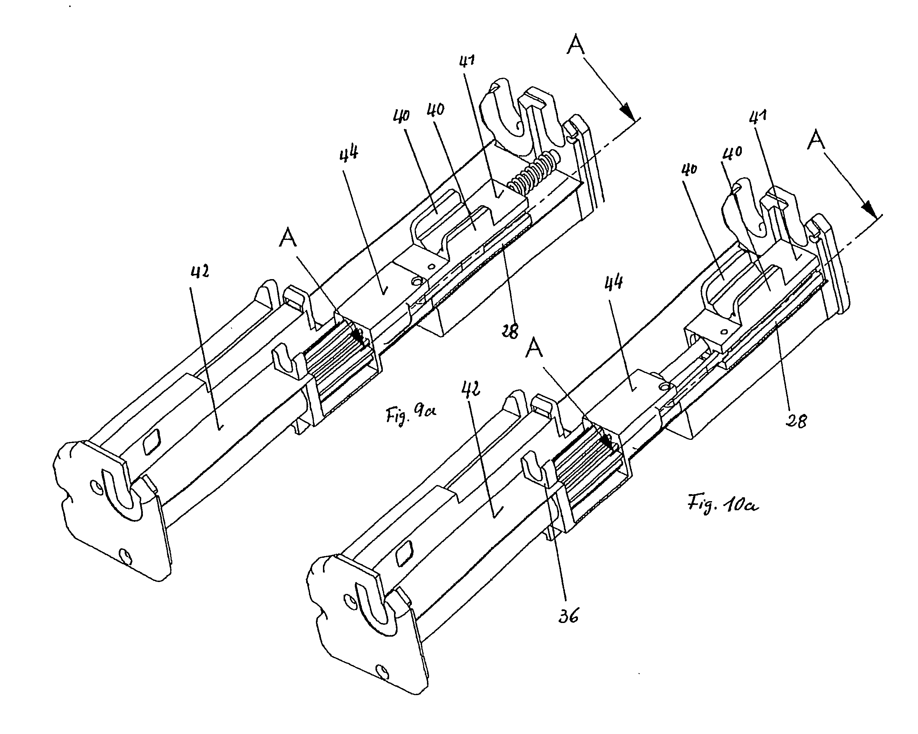

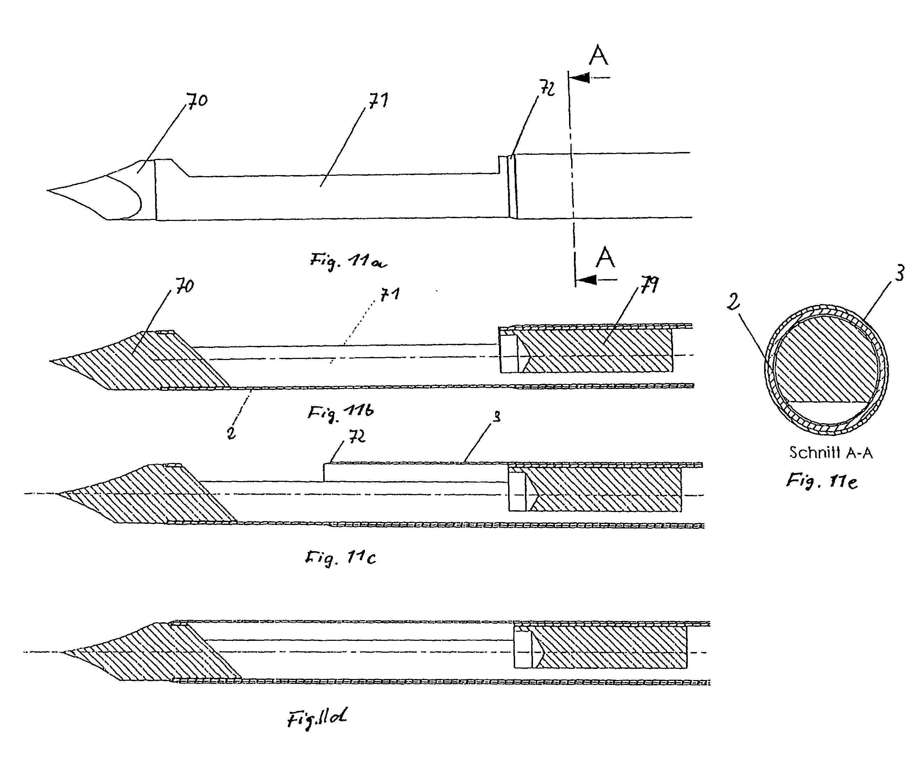

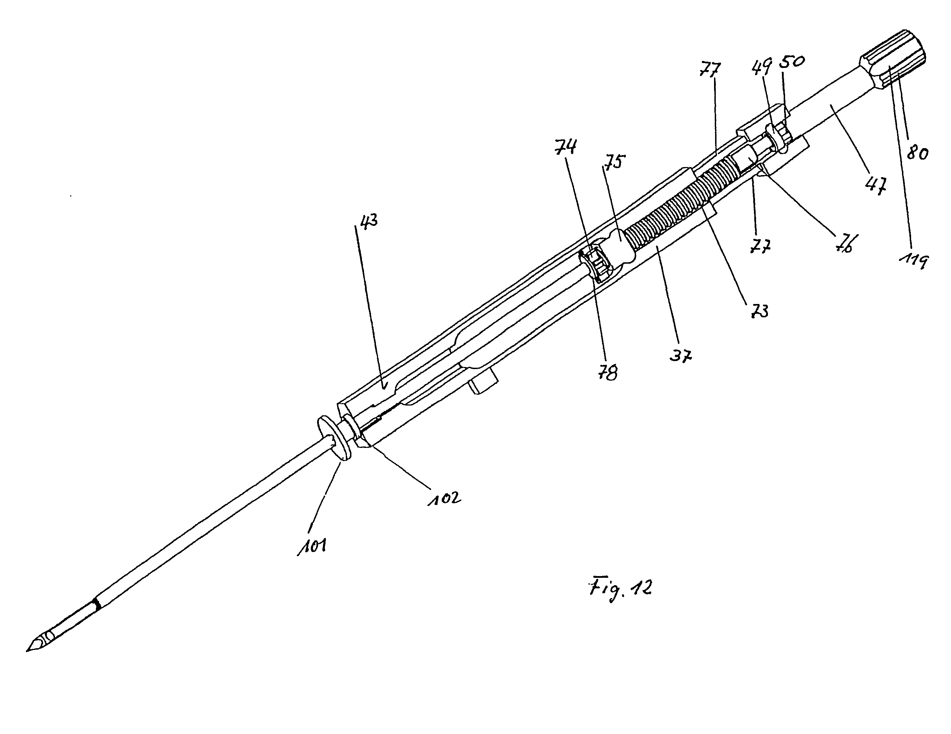

- Fig. 7: Platinenvorderseite
- Fig. 8a: Basisblock in X-Achse gesehen von vorne (perspektivisch)
- Fig. 8b: Basisblock in X-Achse gesehen von hinten (perspektivisch)
- Fig. 9a: Gehäusefeste Einheiten der Biopsievorrichtung ohne Gehäusedeckel und Boden im ungespannten Zustand
- Fig. 9b: Verrasteinrichtung im ungespannten Zustand
- Fig. 10a: Wie Fig. 9, jedoch Spannschlitten in gespannter Position
- Fig. 10b: Wie Fig. 9a, jedoch in verriegeltem Zustand
- Fig. 11a: Biopsienadelspitze Seitenansicht
- Fig. 11b: Längsschnitt durch Fig. 11a (Probeentnahmeraum geöffnet)
- Fig. 11c: Wie Fig. 11b, jedoch (Probeentnahmeraum halb geöffnet)
- Fig. 11d: Wie Fig. 11b (Probeentnahmeraum mittels Schneidhülse verschlossen)
- Fig. 11e: Schnitt A - A in Fig. 11a
- Fig. 12: Biopsienadelträger mit eingepresster Biopsienadel/Schneidhülse und Kunststoffteil (von unten, um ca. 90° gedreht, perspektivisch)
- Fig. 13: Vakuum-/Druckvorrichtung, Einbau und Antrieb (von hinten gesehen, perspektivisch)
- Fig. 14a: Vakuum-/Druckvorrichtung mit auf den Spritzenboden aufgesetzten Kolben (Ausgangsstellung für Vakuumerzeugung und Endstellung für Druckerzeugung, teilweise aufgeschnitten)
- Fig. 14b: Vakuum-/Druckvorrichtung mit zurückgezogenem Kolben; Endstellung des Vakuumhubs (teilweise aufgeschnitten)
- Fig. 14c: Freigabe der Belüftungsbohrung;(Spritzenkolben über Belüftungsbohrung zurückgefahren; Druckausgleichsstellung, teilweise aufgeschnitten)
- Fig. 14d: Schnitt A - A durch die Gewindespindel in Fig. 14c
- Fig. 15: Basisblock und Biopsienadel/Schneidhülse, vorbereitet für die Bestückung mit Fotozellen und Mikroschalter für die IstWert-Erfassung
- Fig. 16: Einlegeelement abgehoben von der Einlegehilfe (perspektivisch)
- Fig. 17: Einlegehilfe (perspektivisch)
- Fig. 18: Koaxialkanüle und Dorn (Explosionsdarstellung)
- Fig. 19: Schnitt durch die Kappe einer Koaxialkanüle
- Fig. 20: Koaxialkanüle mit eingesetzter Nadeleinheit
- Fig. 21: Koaxialkanüle mit eingesetzter Nadeleinheit unter Verwendung eines Distanzstücks

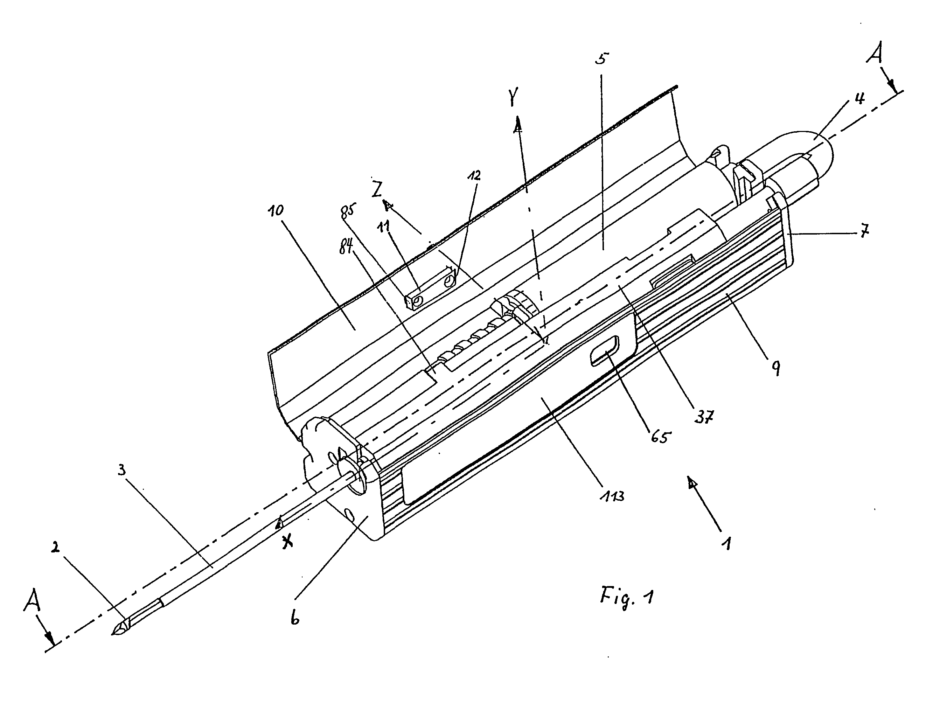

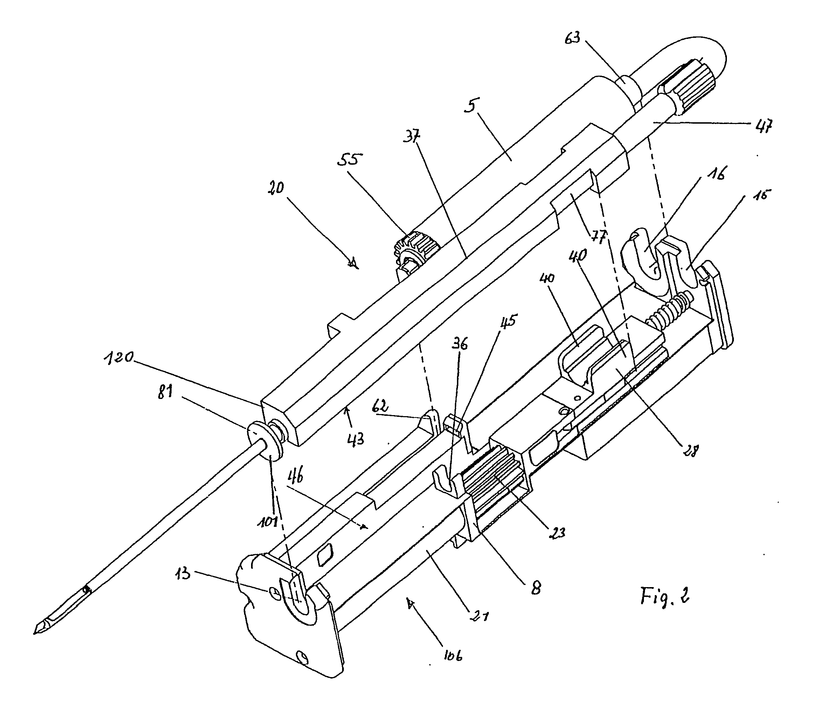

Im Gehäuseinnenraum eines Handstückes 1 sind alle für die Durchführung einer Vakuumbiopsie erforderlichen Vorrichtungen integriert (Fig.1), so dass keine Kabel oder Leitungen vom Gehäuse des Handstücks zu weiteren externen Versorgungsvorrichtungen erforderlich sind. Das Handstück 1 stellt somit eine komplette Vakuumbiopsievorrichtung dar, die nach allen Richtungen frei beweglich ist. Aus dem distalen Teil des Gehäuseenddeckels 6 ragt der distale Teil der hohlen Biopsienadel 2 mit der sie koaxial umgebenden Schneidhülse 3 heraus, der für die Entnahme der Gewebeprobe erforderlich ist. Meist wird eine Koaxialkanüle in das Gewebe gesetzt, in die die Biopsienadel 2 mit Schneidhülse 3 eingebracht wird. Außerhalb des rechten Gehäuseenddeckels 7 ist ein Verbindungselement 4 geführt, z.B. ein durchsichtiger, flexibler Schlauch, der die parallel zur Biopsienadel angeordnete Vakuum/Druckerzeugungsvorrichtung 5 mit dem Innenhohlraum der Biopsienadel 2 verbindet. Das hohle Verbindungselement 4 ist in unmittelbarer Nähe des Gehäuseenddeckels 7 angeordnet. Die in einem Biopsienadelträger 37 angeordnete Biopsienadel mit Schneidhülse und weiteren Elementen bildet mit dem Verbindungselement 4 und der Vakuum-/Druckerzeugungsvorrichtung 5 ein leicht nach oben herausnehmbares sowie einlegbares Element 20, das nach Bedarf gewechselt wird (Fig. 2). Hierzu wird der Gehäusedeckel 10 geöffnet. Wie insbesondere Fig. 2 zeigt, lässt sich die Biopsievorrichtung in Teile, die fest mit dem Gehäuse verbunden sind (desinfizierte Teile), und in ein herausnehmbares Element 20 (steriler Teil)) gliedern. Während die mit dem Gehäuse fest verbundenen Teile lediglich desinfiziert werden, wird das herausnehmbare Element 20 steril verpackt angeliefert und je nach Bedarf , vor allem bei jedem neuen Patienten, erneuert.
Im nachfolgend beschriebenen Ausführungsbeispiel ist die Vakuum/Druckerzeugungsvorrichtung parallel zur Biopsienadel angeordnet. Im Rahmen der Erfindung kann jedoch die Vakuum-/Druckerzeugungsvorrichtung auch in der Achse der Biopsienadel oder des Handstücks liegend angeordnet sein; auch bedarf es keines eigenen Verbindungselementes, wenn sie z.B. unmittelbar auf das Ende der Biopsienadel aufgesetzt ist.

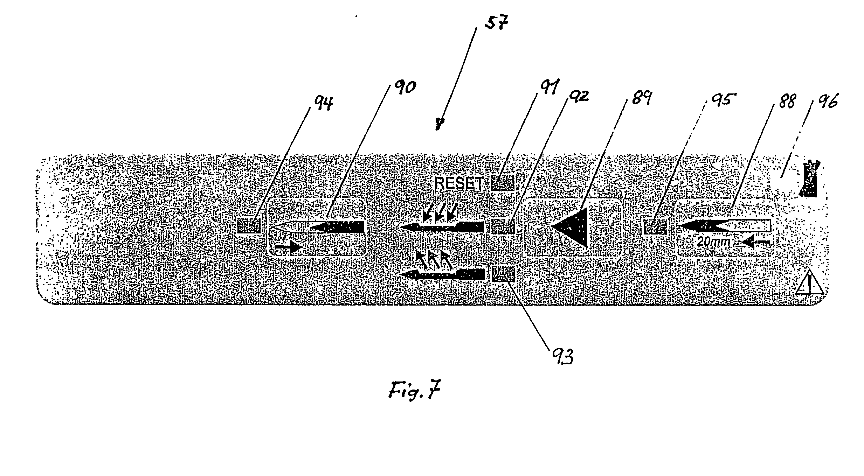

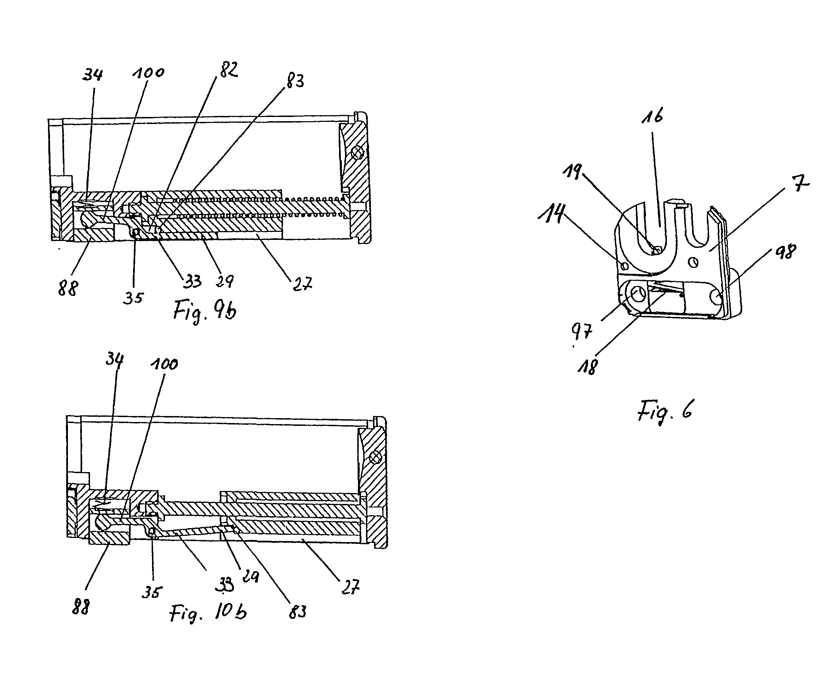

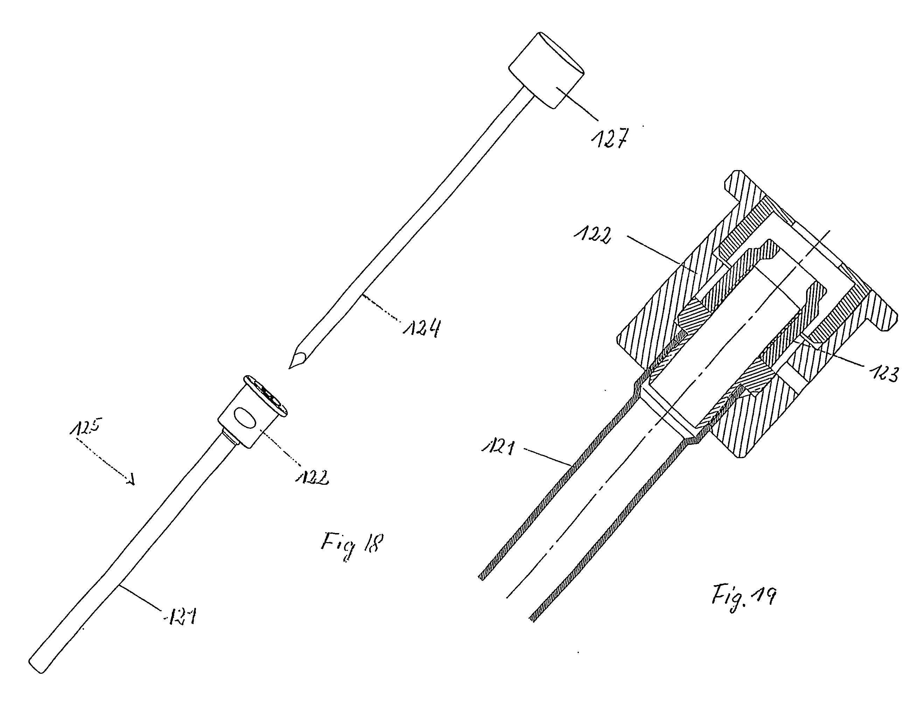

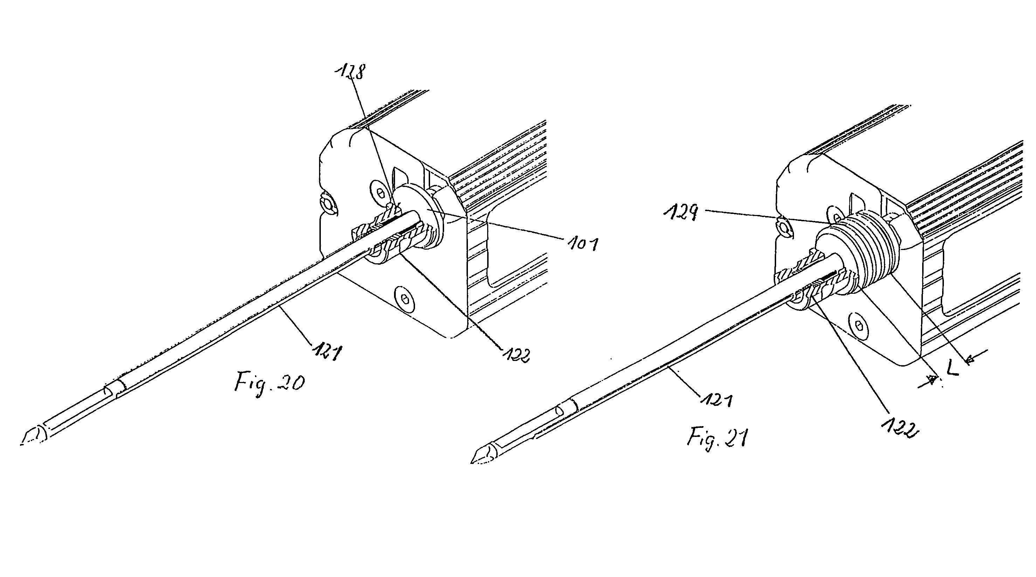

Zwischen dem linken und dem rechten Gehäuseenddeckel 6, 7 befindet sich ein Gehäuseunterteil 9 und ein in den Gehäuseenddeckeln schwenkbar gelagerter Gehäusedeckel 10 mit einem Verschlussriegel 11. Über Zuganker oder Schrauben, die teilweise in einen Basisblock 8 eingeschraubt sind, wird der Gehäuseunterteil 9 zwischen die Gehäuseenddeckel 6, 7 eingeklemmt, bzw. mit dem Basisblock 8 verbunden. Der Gehäusedeckel 10 ist über eine in den Gehäuseenddeckein 6, 7 befestigte Achse schwenkbar verbunden. Der Gehäusedeckel 10 wird vor dem Betrieb der Biopsieeinrichtung geschlossen; die Innenkontur des Gehäusedeckels entspricht der Außenkontur des später genauer beschriebenen Biopsienadelträgers 37. Etwa im Zentrum des Gehäuseinnenraumes, ist der Basisblock 8 angeordnet, der mit dem Gehäuseunterteil z.B. über Fixierelemente und/oder über eine Schraubverbindung fest verbunden ist. Mit dem Basisblock 8, der sich nicht nur in der Längsachse von der Mitte nach links, sondern auch über die gesamte Querfläche erstreckt, sind die Antriebselemente für die Vakuum-/Druckerzeugungsvorrichtung 5, die Schneidhülse 3 und die Spanneinrichtung für den Spannschlitten 28, auf dem der Biopsienadelträger 37 aufgesetzt ist, verbunden. Weiterhin weist der Basisblock 8 eine nach oben offene Halterung 36 für die Biopsienadel/Schneidhülse und ein weiteres Einlegeelement 62 für die Vakuum-/Druckerzeugungsvorrichtung auf.
Zur Beschreibung der Lage der einzelnen Elemente, sowie der Lage der Einzelteile, insbesondere im Gehäuseinnenraum, wurde in Fig. 1 ein Koordinatenkreuz eingezeichnet, wobei der Koordinatenmittelpunkt des Koordinatensystems im Zentrum des Basisblocks 8 (Fig.1) liegt. Danach gelten für die nachfolgende Beschreibung und für die Ansprüche für die Richtungsangaben in Richtung der X-Achse gesehen als links (distal) und entgegengesetzt der X-Achse gesehen als rechts (proximal). Für die übrigen Koordinaten gilt in Richtung der Y-Achse als oben, entgegensetzt der Y-Achse als unten und in Richtung der Z-Achse als hinten und entgegengesetzt der Z-Achse als vorne (Fig. 1). Das Koordinatensystem teilt den Geäuseinnenraum und die übrigen Bezüge also in links und rechts, in vorn und hinten sowie oben und unten auf.
Unter Bezug auf diese Festlegungen befinden sich etwa im unteren, vorderen, linken Gehäuseteil des Gehäuseinnenraumes die gemeinsamen Antriebseinrichtungen 106 für die Spanneinrichtung und die Schneidhülse sowie im unteren, hinteren, linken Gehäuseteil die Antriebseinrichtung 105 (Fig. 13) für die Vakuum/Druckerzeugungsvorrichtung 5. Im unteren, rechten Teil ist die Energieversorgung für die Antriebsmotoren und die sonstige Elektrik untergebracht, wie z.B. für die Steuer- und/oder Überwachungselemente; vorzugsweise werden hierfür Batterien oder ein Akku 111, z.B. eine 7,2V-Lithium-lonen-Batterie, 1Ah verwendet. Der vordere, rechte, obere, über dem Batterieraum liegende Gehäuseinnenraum wird weitgehend für den Spannschlitten 28 mit Verriegelungsteil genutzt (Fig. 5); der mit einem Block 26, der Teil des Basisblocks 8 ist, verbunden ist. Der Batterieraum ist nach oben durch eine Trennplatte 114 abgedichtet.
Im obersten, vorderen Teil des Gehäuseinnenraums ist ein in die U-förmige, nach oben offene Einlegehalterung 36 des Basisblocks 8 und in die nach oben zeigenden, beidseitig des Spannschlittens 28 angeordnete Lasche 40 einlegbarer und herausnehmbarer Biopsienadelträger 37, in dem die Biopsienadel/Schneidhülseneinheit mit Antriebsteilen drehbar gelagert ist, der über nahezu die gesamte Länge des Handstücks reicht, angeordnet. Der Biopsienadelträger ist, wie später beschrieben, mittels des Spannschlittens längsverschieblich. Dies bedeutet, in nicht gespanntem Zustand liegt die linke Stirnfläche des Biopsienadelträgers 37 am linken Gehäuseenddeckel 6 an, in gespanntem Zustand die rechte Stirnfläche am rechten Gehäuseenddeckel 7. "Nahezu der gesamten Länge" bedeutet, dass der Biopsienadelträger mindestens um den Betrag kürzer ist, als der Gehäuseinnenraum für den Spannvorgang benötigt wird. Beträgt der Spannweg des Spannschlittens z.B. 20 mm so muss sich der Biopsienadelträger um diesen Betrag verschieben lassen. Im Allgemeinen liegt der Spannweg zwischen 15 und 25 mm, je nach verwendeter Biopsienadel. Daher ist es zweckmässig, den Innenraum auf den größtmöglichen Spannweg auszulegen.
Die Spannvorrichtung (rechts, vorn liegend) selbst, besteht aus einem auf einem Bolzen 30 geführten Spannschlitten 28, wobei der Bolzen in den Basisblock 8 einschraubbar ist. Der Bolzen 30 wird von einer Spiralfeder 31 umgeben. Die Verrastvorrichtung (sh. insbesondere Fig. 9b und 10b) des Spannschlittens ist an dem Block 26 befestigt . Im oberen, hinteren, rechten Gehäuseinnenraum ist die Vakuum/Druckerzeugungsvorrichtung 5 mit Teilen des Antriebs untergebracht; der Antriebsmotor mit Reduktionsgetriebe für die Vakuum-/Druckerzeugungsvorrichtung befindet sich im linken, unteren, hinteren Bereich des Gehäuseinnenraumes.
Das Handstück wie auch insbesondere die Biopsienadel oder die Vakuum/Druckerzeugungsvorrichtung sind weder über Kabel oder Schlauchleitungen mit außerhalb des Gehäusehandstücks liegenden zusätzlichen Versorgungseinheiten verbunden. Die Beweglichkeit und Manövrierfähigkeit wird daher weder durch Leitungen noch durch Kabel eingeschränkt.
Der Gehäusedeckel, das Gehäuseunterteil, die Gehäuseenddeckel sowie der Basisblock bestehen vorzugsweise aus Aluminium.
Das Handstück 1 besteht, wie bereits beschrieben, aus einem Gehäuse, das aus einem Gehäuseunterteil 9 mit seitlich unterschiedlich hochgezogenen Wänden, dem Gehäuseunterteil angepassten Gehäusedeckel 10 mit längsverschieblichem Verschlussriegel 11 und den beiden Gehäuseenddeckeln 6, und 7 gebildet wird. Das Gehäuseunterteil ist mit den beiden Gehäuseenddeckein über Zuganker oder Schrauben, z.B. aus Eisen verbunden, die teilweise unmittelbar in den Basisblock 8 eingeschraubt werden. Das Gehäuse ist ca. 200 mm lang, die Gehäuseenddeckel haben etwa quadratischen Querschnitt, ca. 40 x 40 mm, (Fig. 2). Der Gehäusedeckel 10 ist um eine Achse 104 verschwenkbar, die in den Gehäuseenddeckeln 6, 7 befestigt ist; hierzu dienen in den Gehäuseenddeckein die Bohrungen 14. Die Nase 12 des Verschlussriegels 11 ist in die Ausnehmung 45 des Basisblocks 8 zum Verschließen des Gehäusedeckels einschiebbar. Der linke Gehäuseenddeckel 6 weist im oberen, vorderen Teil eine nach oben offene U-förmige Durchführung 13 für den nach vorne herausragenden Teil der Biopsienadel/Schneidhülse 2, 3 und der darauf angeordneten Führungsrolle 81 auf. Der hintere Gehäuseenddeckel 7 weist zwei nach oben offene, U-förmige Durchführungen 15, 16 auf. Die Durchführung 15 korrespondiert mit der Durchführung 13; sie nimmt das Ende des auf die hohle Biopsienadel aufgesetzten querschnittsrunden Kunststoffteils 47 auf. In die Durchführung 16 wird ein Stutzen 63 der Vakuum/Druckerzeugungsvorrichtung eingelegt (Fig. 2). Ein in das Kunststoffteil 47 eingesetztes, weiteres Kunststoffteil 112 weist einen Zapfen 17 auf, der zur Verbindung des Verbindungselements 4 mit dem Ausflussstutzen 64 der Vakuum/Druckerzeugungsvorrichtung dient. Der Innenhohlraum der Biopsienadel ist über das ebenfalls hohle Verbindungselement 4 durchgängig mit dem Hohlraum der Kolben/Zylinderanordnung und dem Hohlraum der Vakuum-/Druckerzeugungsvorrichtung verbunden. Die Verbindungen sind derart gestaltet, dass in das System weder Luft von außen eindringen kann, noch bei Überdruck Luft nach außen ausströmen kann; die Verbindungsstellen sind also luftdicht. Wie insbesondere Fig. 6 zeigt, ist in die Durchführung (16) des Gehäuseenddeckels 7 auf der unteren Seite ein Mikroschalter 18 integriert, dessen Schaltstift 19 in die Durchführung hineinragt.
Sobald der Stutzen 63 der Vakuum-/Druckerzeugungsvorrichtung in die Durchführung eingelegt wird und der Gehäusedeckel geschlossen ist, wird der Schaltstift 19 des Mikroschalters 18 nach unten gedrückt und der Mikroschalter 18 gibt die Stromzufuhr frei. In die Durchführungen 97, 98 des Gehäuseenddeckels können die Anschlüsse zum Anschluss eines Ladegerät eingebaut werden.
An der vorderen Seite des Gehäuseunterteils 9 ist eine Fläche 113 für die zu integrierende Platine mit den Bedien- und Überwachungselementen vorgesehen (Fig. 1). Die am Gehäuse zu befestigende Platine 57 ist als eigenes Bauteil ausgebildet, das beispielsweise auf die Fläche 113 des Gehäuseunterteils 9 aufgeklebt wird. Über Leitungen ist diese Platine 57 mit weiteren elektronischen Bauteilen, die im Gehäuse angeordnet sind, sowie mit der Stromversorgung verbunden. Die Platine, beinhaltet vor allem Schalter zum Bedienen und Dioden für die Kontrolle. Aus einer Aussparung 65 im Gehäuseunterteil und der Platine ragt die Betätigungstaste 88 für die mechanische Auslösung des gespannten Spannschlittens heraus;
Bei der Gestaltung der Bedien- und Überwachungselemente wurde darauf geachtet, dass zwischen dem Spannvorgang des Spannschlittens und dem Auslösen des Spannschlittens einerseits, und andererseits der Durchführung der Biopsie, wie dem Heraustrennen der Probe, sowie der Probeentnahme mit dem Auswurf der Probe unterschieden wird. Dementsprechend ist die Betätigungstaste 88 (Auslöser) für den Spannschlitten nach rechts und die das Spannen des Spannschlittens auslösende Spanntaste 90 nach links gesetzt worden. Die für das Durchführen der Biopsie vorgesehene Programmtaste 89 liegt mittig. Das Drücken der Programmtaste ist für drei Funktionen erforderlich. Die erste Funktion, Beginn, bzw. Wiederholung (Reset) zeigt die Resetdiode 91 an, die darunter angeordnete Probeentnahmediode 92 zeigt das Öffnen und Schließen des Probeentnahmeraumes bei der Probeentnahme an. Die unterste Auswurfdiode 93 zeigt das Auswerfen der entnommenen Probe an. Die Spanndiode 94 zeigt das Spannen des Spannschlittens an; die Verriegelungsdiode 95 zeigt das Verriegeln des Spannschlittens an. Die Batterieladediode 96 zeigt den Ladungszustand der Batterie, bzw. der Akkus an. Die Dioden sind so geschaltet, dass bei Durchführung des aufgerufenen Vorgangs die Diode blinkt und nach Abschluss des Vorgangs die Diode des Folgevorgangs aufleuchtet. Sofern zwei Möglichkeiten zur Auswahl stehen, leuchten beide Folgedioden auf. Es ist dem Bediener dann freigestellt, welche Auswahl er trifft. Auf die Wirkungsweise und Steuerungsmöglichkeit im Einzelnen wird bei der Beschreibung des Ablaufvorgangs näher eingegangen. Symbole (Pictogramme) auf dem Bord symbolisieren die einzelnen Vorgänge.

Zur Erhöhung der Bediensicherheit kann es zweckmäßig sein, einzelne automatisierte Ablaufvorgänge mit Verzögerungsschaltung auszustatten. Insbesondere hat sich gezeigt, dass die Vorgänge "Spannen des Spannschlittens" durch Drücken der Spanntaste 90 und "Auswurf der Probe" durch Drücken der Programmtaste 89, zur Erhöhung der Bediensicherheit mit Verzögerungsschaltungen von ca. 1,2 - 1,5 Sekunden versehen werden sollten. Weiterhin wird die Bediensicherheit erhöht, wenn die die einzelnen Vorgänge anzeigenden Leuchtdionden für Vorgänge ausßerhalb und Vorgänge innerhalb des Gewebes verschiedene Farben haben.

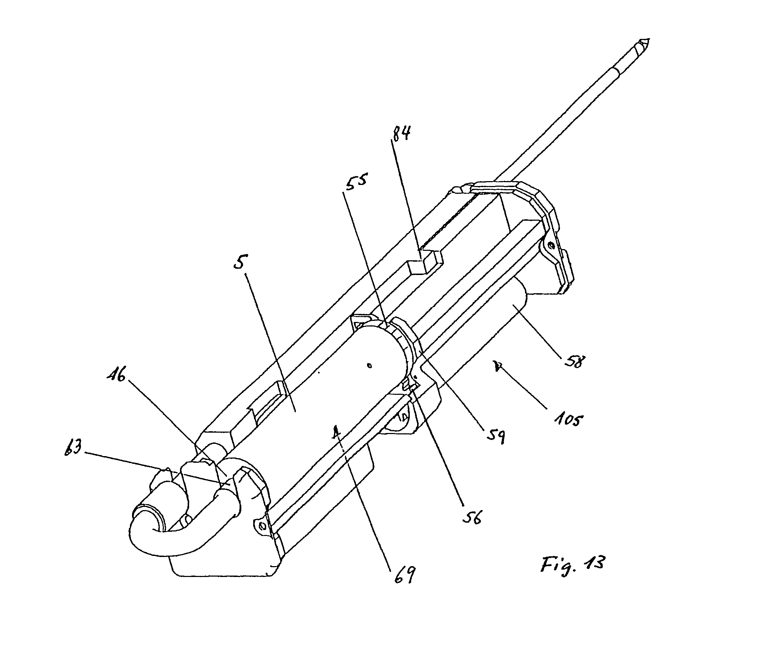

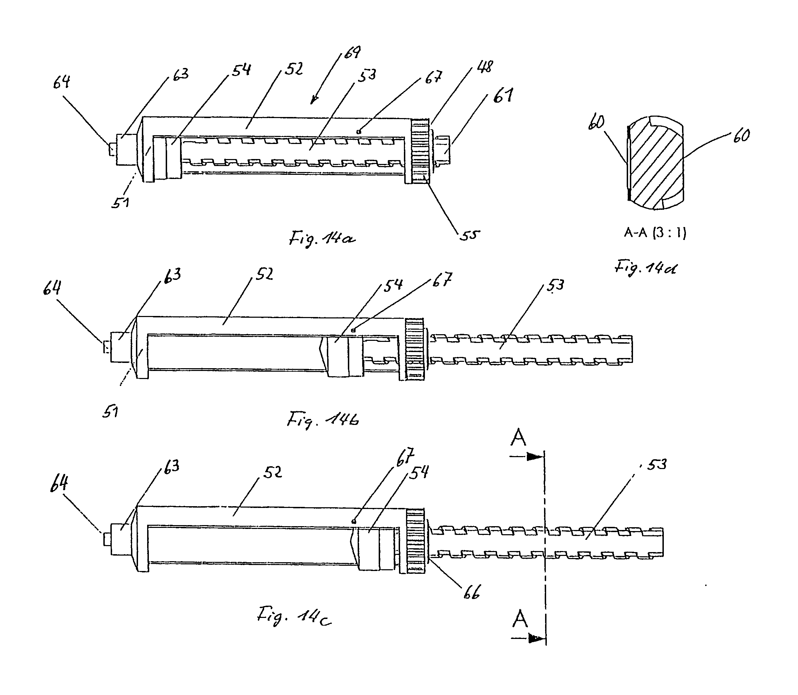

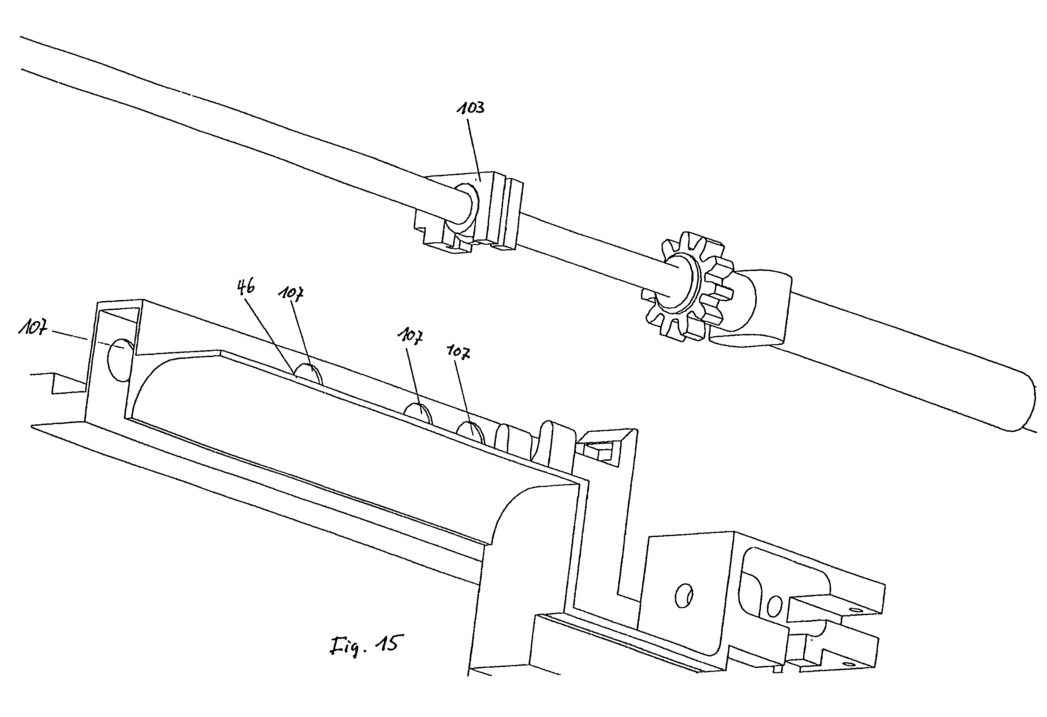

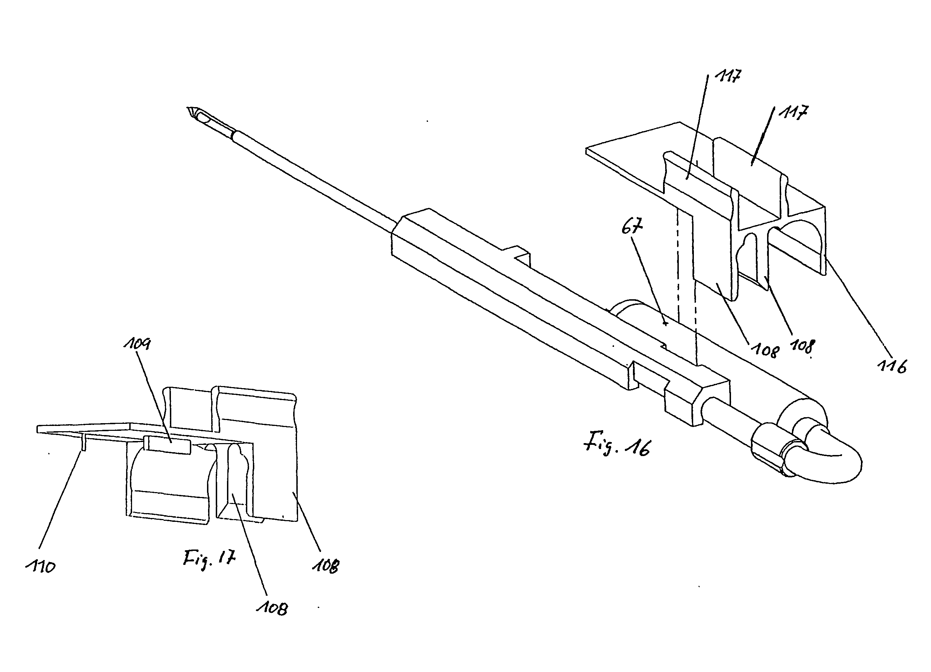

Eine perspektivische Darstellung des Basisblocks 8 (von vorne in Richtung der X-Achse gesehen) zeigt Fig. 8a; den Basisblock 8 von hinten in X-Achse zeigt Fig. 8b (beide Darstellungen perspektivisch). Der Basisblock 8 lässt sich in Längsrichtung gesehen in zwei Hälften gliedern; der vordere Teil dient der Befestigung des gemeinsamen Antriebs für Schneidhülse und Spannschlitten sowie in seinem oberen Teil der Lagerung des Biopsienadelträgers (Fig. 8a); der rückwärtige Teil dient der Befestigung des Antriebs für die Vakuum-/Druckerzeugungsvorrichtung sowie der Lagerung einer Seite der Vakuum-/Druckerzeugungsvorrichtung (Fig. 8b). Zwischen den beiden Antriebsmotoren 21, 58, unter der Mittelrippe 87 ist eine zentrale Elektronikplatine angeordnet. Der Basisblock 8 weist in seinem linken, vorderen Teil einen U-förmigen Raum 24 auf, in den eine Zahnwalze 23, die von dem Getriebemotor 21 angetrieben wird, eingebaut ist. Hierzu ist die Abtriebswelle des Getriebemotors in einer Öffnung in der Wand 25 des Basisblocks 8 gelagert bzw. eingesteckt. Die Zahnwalze 23 ist auf die Abtriebswelle aufgesteckt und auf ihr z.B. mittels einer Schraube dreh- und verschiebesicher befestigt. Auf der anderen Seite ist die Zahnwalze 23 in der Wand 22 des Basisblocks 8 gelagert. Als Antriebsmotor wird ein Gleichstrommotor mit einer Drehzahl von ca. 11000 U/min verwendet. Dem Gleichstrommotor ist ein Planetengetriebe mit hoher Untersetzung nachgeschaltet, auf dessen Abtriebswelle ist die Zahnwalze 23 aufgesetzt.
An der Wand 22 ist nach rechts zeigend ein weiterer Block 26 angeformt, der sowohl den verschwenkbaren Doppelhebel 33 für die Verriegelung aufnimmt, als auch zur Befestigung des Bolzens 30 für die Führung des Spannschlittens 28 dient. Der Bolzen 30 wird in die Gewindebohrung 29 eingeschraubt. Der Spannschlitten 28 gleitet beim Spannvorgang auf dem Bolzen 30 und der darunter angeordneten Trennplatte 114 nach rechts. Beim Spannvorgang wird die auf dem Gewindebolzen 30 angeordnete Spiralfeder 31 zusammengedrückt. Die Spiralfeder stützt sich mit einem Ende auf einem Endstück 32 des Gewindebolzens oder direkt am Gehäuseenddeckel 7 ab; das andere Spiralfederende stützt sich am Ende der Führungsbohrung 115 des Spannschlittens ab.
Der Spannschlitten 28 gleitet auf den Gewindebolzen und der Trennplatte 114 und ist damit verdrehgesichert. Ein Arm 99 des doppelarmigen Hebels 33 der Verrrastvorrichtung greift in eine Nut 27 des Spannschlittens 28 ein (Fig. 9a und 10a). Die in den Block 26 des Basisblocks 8 integrierte Verrastvorrichtung besteht aus dem doppelarmigen Hebel 33, der um eine senkrecht stehende Achse 35 (in Y-Achse gesehen) mittels einer Druckfeder 34 verschwenkbar ist. Die Achse 35 , ein senkrecht stehender Stift, ist in den Bohrungen 38 des Basisblocks befestigt. Im ungespannten Zustand liegt der Teil 99 des doppelarmigen Hebels in der Nut 27 des Spannschlittens; die zusammengedrückte Feder 34 wirkt auf den Teil 100 des Hebels ein um die Verrasttaste 88 nach außen (nach vorn) zu drücken. Sobald der Teil 99 des doppelarmigen Hebels in die Ausnehmung 82 des Spannschlittens einrasten kann, wird die Betätigungstaste 88 nach außen gedrückt. Der Spannschlitten wird durch Einrasten des Hebelteils 99 im gespannten Zustand verrastet und kann nun bei Bedarf mit der Betätigungstaste 88 ausgelöst werden. Da der Spannschlitten zweckmäßigerweise aus Kunststoff gefertigt ist, hat es sich als zweckdienlich erwiesen, in die Vertiefung ein Metallteil 83 einzusetzen um den Kunststoff nicht zu beschädigen, da ja der doppelarmige Hebel aus Metall gefertigt ist. Im Gegensatz zum herausnehmbaren Element 20 wird das Handstück mit erneuertem Einlegeelement mehrfach verwendet. Der Spannweg entspricht der Eindringtiefe der Biopsienadel in das Gewebe. Daraus ergibt sich, dass die Länge des Hebels 99 ebenfalls dem Spannweg entspricht. Da die Eindringtiefen in der Regel zwischen 15 und 25 mm liegen, kann durch entsprechende Ausbildung der Länge des Hebels 99 und entsprechender Vorgabenänderung in der Steuerung, das gleiche Handstück für verschiedene Eindringtiefen verwendet werden.

Der sich an den Block 26 anschließende Spannschlitten 28 ist höhengleich angeordnet und ist etwa querschnittgleich. An seiner oberen Seite weist der Spannschlitten zwei Laschen 40 auf. Die nach oben zeigende Fläche 41 des Spannschlittens, sowie die nach oben zeigende Fläche 44 des Blocks 26, sowie die nach oben zeigende Fläche der Verlängerung 42 des Basisblocks 8, bilden zusammen eine plane Lagerfläche für die untere Gleitfläche 43 des aufzusetzenden Biopsienadelträgers 37. Der Biopsienadelträger ist aus Kunststoff gefertigt. Bei der Verschiebung des Spannschlittens vom ungespannten Ausgangszustand (Fig. 9a) in den gespannten Zustand (Fig. 10a), also nach rechts, gleitet der von den Laschen 40 gehaltene Biopsienadelträger 37 über die Fläche 42 und 44. Es ist auch denkbar, dass die Gleitflächen nicht plan, wie beim Ausführungsbeispiel gestaltet sind, sondern eigens gestaltete Gleitflächen aufweisen; wichtig ist, dass der Biopsienadelträger 37 auf der Gleitfläche leichtgängig und geradlinig gleiten kann und dass nach dem Auslösen der Betätigungstaste 88 die Biopsienadel geradlinig in das Gewebe, den Tumor, eindringen kann. Deshalb ist auch die obere Außenkontur des Biopsienadelträgers der Innenkontur des Gehäusedeckels entsprechend ausgebildet und weist ein nur geringes Spiel zum Gehäusedeckel auf um ein Ausweichen der Biopsienadel nach oben zu verhindern.

Oberhalb des U-förmigen Raumes 24 für die Zahnwalze 23, in der Höhe der Gleitfläche 42, weist der Basisblock 8 eine U-förmige, nach oben offene Halterung 36 u.a. für das Einlegen der Biopsienadel/Schneidhülse auf. Diese Halterung dient vor allem als radiales Drucklager, also zur Abstützung für den mit der Schneidhülse verbundenen Antriebsteil, das Zahnrad 74, bzw. die Kunststoffscheibe 78 um den Spannschlitten mittels der Antriebsvorrichtung 106 in seine Spannposition zu bringen, wie später beschrieben wird.

Im hinteren, oberen Teil des Basisblocks ist ein weiteres U-förmiges Einlegeelement 62 vorgesehen, in das das aus dem Spritzenkörper herausragende freie Ende 61 der Gewindespindel der Vakuum-/Druckerzeugungsvorrichtung eingelegt wird. In der Mitte, oben, des Basisblocks ist eine Befestigung für eine Platte, die die Ausnehmung 45 aufnimmt, vorgesehen, in die die Nase 12 des Verschlussriegels 11 des Gehäusedeckels eingeschoben wird. Eine am Basisblock 8 angeordnete Abdeckung 46, die nach links zeigt, trennt den Raum der Antriebsmotoren und der eingesetzten Platine, von dem oberen, linken Teil des Gehäuseinnenraums, der vor allem der Lagerung des austauschbaren Biopsienadelträgers 37, einschließlich Biopsienadel und Schneidhülse dient. Die Abdeckung 46 schützt die elektrischen Getriebemotoren und die Platine vor Verschmutzung. Die Platine für die Elektronik liegt zwischen den Antriebsmotoren und unterhalb der Mittelrippe.
Den Biopsienadelträger 37, der in die Laschen 40 des Spannschlittens 28 mit Biopsienadel 2 und Schneidhülse 3 sowie weiteren Teilen einlegbar ist, zeigt Fig. 2. Die hohle, kreisrunde Biopsienadel 2 hat eine Nadelspitze 70, an die sich der Probeentnahmeraum 71 anschließt (Fig. 11a - 11e). Die querschnittsrunde Biopsienadel 2 wird von einer koaxial angeordneten, querschnittsrunden Schneidhülse 3 umgeben, die an ihrem linken, dem Probeentnahmeraum zugewandten Ende eine Schneide 72 aufweist, die dazu dient, nach Einführung der Biopsienadel (mit geschlossenem Probeentnahmeraum) und nach Öffnen des Probeentnahmeraums und Einsaugen der Probe in den Probeentnahmeraum, die Gewebeprobe herauszuschneiden. Die Schneide steht vorzugsweise senkrecht zur Längsachse von Biopsienadel und Schneidhülse. Der Abtrennvorgang geschieht durch Rotation und gleichzeitiger Längsverschiebung der Schneidhülse mittels des Gewindespindelantriebs. Es ist auch denkbar, dass die Bewegung nicht kontinuierlich erfolgt, sondern schrittweise oder vibirerend, d.h. der Vorschub wird in kurzen Abständen vor und zurück bewegt.

Auf dem der Schneide 72 abgewandten anderen, proximalen Ende der Schneidehülse ist eine Gewindespindelhülse 73 mit einem auf der Stirnseite der Gewindespindelhülse angeordneten Zahnrad 74 befestigt. Die Gewindespindelhülse mit Zahnrad ist auf der Schneidhülse dreh- und verschiebesicher angeordnet. Mit der Gewindespindel arbeitet eine Gewindespindelmutter 75 zusammen, die fest in den Biopsienadelträger 37 eingepresst ist. Das Zahnrad 74 liegt links ,also vor dem Beginn der Spindelhülse. Bei Verdrehen der Gewindespindelhülse mittels des Zahnrades 74 wird die Schneidhülse gedreht und in Längsrichtung über der Biopsienadel 2 verschoben.
Das Zahnrad 74 am linken Ende der Gewindespindel kämmt nach dem Einsetzen des Biopsienadelträgers in die Laschen 40 mit der Zahnwalze 23. Um den Biopsienadelträger 37 bei nicht gespanntem Spannschlitten (Fig. 2) in die Laschen des Spannschlittens einsetzen zu können, weist der Biopsienadelträger zwei plane, parallele Ausnehmungen 77 auf. Beim Aufsetzen der Gleitfläche des Biopsienadelträgers 37 auf die Flächen 41, 42 und 44 wird gleichzeitig die Schneidhülse in die Halterung 36 des Basisblocks 8 eingesetzt. Auf der linken Seite des Zahnrades kann zur Verbesserung der Drehbarkeit des Spindelantriebs, insbesondere, wenn die Halterung 36 als Abstützung für das Spannen des Spannschlittens dient, eine Kunststoffscheibe 78 eingefügt sein, die mit einem leichten Konus versehen ist. Bei korrekt eingelegtem Biopsienadelträger gleitet beim Spannen des Spannschlittens der Biopsienadelträgers mit der Gleitfläche 43 über die Flächen 42 und 41 nach rechts. Da nach dem Einlegen des Biopsienadelträgers zunächst der Probeentnahmeraum geschlossen wird, liegt das Zahnrad 74 an der Halterung 36 an. Wird nun die Zahnwalze 23 weiter in gleicher Richtung angetrieben, so schraubt der Gewindespindelantrieb über den Biopsienadelträger den Spannschlitten nach rechts, bis er verrastet ist; dabei wird die Biopsienadel nach innen gezogen, während die Schneidhülse in ihrer Position verbleibt. Die Schneidhülse ragt nach dem Verrasten über die Biopsienadelspitze hinaus. Es wird daher nach Verrastung des Spannschlittens die Schneidhülse in die Ausgangslage (entgegengesetzte Drehrichtung) zurückgedreht; das Zahnrad 74 verschiebt sich hierbei in der Zahnwalze von links nach rechts. Nach dem Entrasten des Spannschlittens gleitet mit dem Biopsienadelträger die Biopsienadeladel-/Schneidhülse mit Zahnrad wieder nach links. Nun kann die Schneidhülse wieder nach rechts verschoben werden um den Probeentnahmeraum zu öffnen.

Das rechte Ende der Schneidhülse ist über ein Dichtelement 76 mit der hohlen Biopsienadel rotationsbeweglich, aber luftabschließend verbunden, damit weder Luft zwischen Biopsienadel und der sie koaxial umgebenden Schneidhülse eindringen, noch bei Überdruck Luft austreten kann. Auf das rechte Ende der Biopsienadel 2 ist ein rundes, ebenfalls hohles Kunststoffteil 47 luftdicht aufgesetzt und mit der Biopsienadel kraftschlüssig verbunden. Das Kunststoffteil 47 hat an seinem linken Ende ein Lagerelement 49, das in den Biopsienadelträger eingepresst ist; an seinem aus dem Handstück herausragenden rechten Ende ist ein weiteres Kunststoffteil 112 eingesetzt, das gegenüber dem Kunststoffteil 47 und gegenüber der Biopsienadel 2 drehbeweglich ist. Zwischen Biopsienadel und Kunststoffteil 112 ist ein O-Ring zur Abdichtung eingesetzt. Das Kunststoffteil hat an seinem rechten Ende einen Zapfen 17, auf den das Verbindungselement 4 luftdicht aufgeschoben wird. Ebenfalls am rechten, aus dem Biopsienadelträger und dem Gehäuse herausragenden Teil, befindet sich eine Rändelscheibe 80, mit der durch Drehen die Lage des Probeentnahmeraums radial verstellt werden kann, ohne dass die Position der Schneidhülse verändert wird. Mit einer Verdrehung der Biopsienadel ist allein eine Verdrehung des Probeentnahmeraums und damit der Probeentnahmevorrichtung verbunden. Das Kunststoffteil 47 mit Biopsienadel und Schneidhülse wird mit dem Lagerelement 49 und der Gewindespindelmutter 75 in den Biopsienadelträger eingepresst. Die Biopsienadel ist über das Lagerelement 49 und seine enge Führung in der Schneidhülse drehbeweglich im Biopsienadelträger und in der Schneidhülse gelagert und mit dem Biopsienadelträger in der Längsachse verschiebbar. Wie vorher beschrieben, ist die Schneidhülse gegenüber der Biopsienadel durch Verdrehen axial beweglich.

Rechts vom Lagerelement 49 ist ein Vielkant 50 auf dem Kunststoffteil angeordnet, der mit dem Biopsienadelträger durch Verspannen verrastbar ist, so kann der Probeentnahmeraum der Biopsienadel mittels der Rändelscheibe 80 in die für die Biopsieentnahme günstigste Position gebracht und gehalten werden.
Einzelheiten des Probeentnahmeraums sowie der Biopsienadelspitze sind in den Fig. 11a -11e dargestellt. Der an die Nadelspitze 70 anschließende Probeentnahmeraum 71 ist etwa über 25% seines Querschnitts nach oben offen. Die Schnittkanten können geschliffen oder angeschärft sein. Der Probeentnahmeraum ist etwa zwischen 15 bis 25 mm lang. Daran schließt sich der Hohlraum der Biopsienadel an. Am Übergang, also am rechten Ende des Probeentnahmeraumes, ist der Hohlraumquerschnitt der Biopsienadel zwischen 50% und 75% durch eine Einengung, z.B. einen Stopfen 79 verschlossen (Fig. 11b - 11e). Die Höhe des Stopfens ist so ausgelegt, dass er über die Ausnehmung des Probeentnahmeraums hinaus nach unten reicht. Das Vakuum soll dadurch vor allem die Gewebeprobe mit dem kontinuierlichen Öffnen des Probeentnahmeraums in den Probeentnahmeraum hineinziehen und an der Wand des Probeentnahmeraums zur Anlage bringen. Bei Überdruck im Biopsienadelhohlraum wirkt die Einengung, der Stopfen, druckerhöhend. Der Stopfen hat etwa die Länge von 10 mm und ist in den Hohlraum eingeklebt oder eingeschweißt. Beim Lasereinschweißen hat sich gezeigt, dass es vorteilhaft ist, die linke Seite des Stopfens durch Herausnahme von Material an der Stirnfläche auf eine kurze Länge, ca. 2 mm, dünn zu gestalten. Dadurch wird in diesem Bereich an der Stirnfläche das Rohr der Biopsienadel mit der Stirnseite des Stopfens durchgeschweißt und ist an der Stirnseite luftdicht. Der Stopfen kann auch von geringerer Länge sein, sofern die gleiche Wirkung erzielt wird. So kann der Stopfen auch durch eine Lippe oder Nase von etwa gleicher Höhe ersetzt werden. Wichtig ist, dass die Verengung so gestaltet ist, dass das Vakuum im Probeentnahmeraum vor allem vom Boden her zur Wirkung kommt, damit die Probe beim Schneidevorgang an der Wand des Probeentnahmeraums anhaftet und ihre Lage nicht verändert. Es hat sich auch als vorteilhaft erwiesen, evtl. zusätzliche Fixiermittel an der Probeentnahmewand vorzusehen. Durch das Einsaugen der Probe von unten in den Probeentnahmeraum ergibt sich zum einen ein hoher Füllungsgrad des Probeentnahmeraums und zum anderen, vor allem durch seine Gestaltung, eine gute Fixierung der Probe an der Wand. Da die Schneidhülse die Probe auf der Außenseite der Biopsienadel abtrennt, bleibt dieses Festsaugen der Probe in der Innenseite auch beim Trennvorgang erhalten. Darüber hinaus wird durch die außen angeordnete Schneidhülse, durch das angelegte Vakuum, kein Gewebe in die hohle Schneidhülse eingesaugt und damit kann das Gewebe nicht durch die rotierende Längsbewegung der Schneidhülse durch Festhalten im Schneidhülseninnenraum verdreht oder verdrillt werden. Die Qualität der Probe wird dadurch verbessert, da der Pathologe ein Ausgangsmaterial erhält, das dem Querschnitt des Schnittes entspricht und nicht verdrillt oder verformt ist. Beim Auswurf der Probe unter Druck findet durch den Stopfen 79 zusätzlich eine vollständige Reinigung des Probeentnahmeraums statt, so dass im Wiederholungsfall keine Vermischung stattfindet. Da die Vakuumerzeugungsvorrichtung gleichzeitig als Druckerzeugungsvorrichtung genutzt wird, wird der gesamte Hohlraum, insbesondere der Nadel gereinigt.

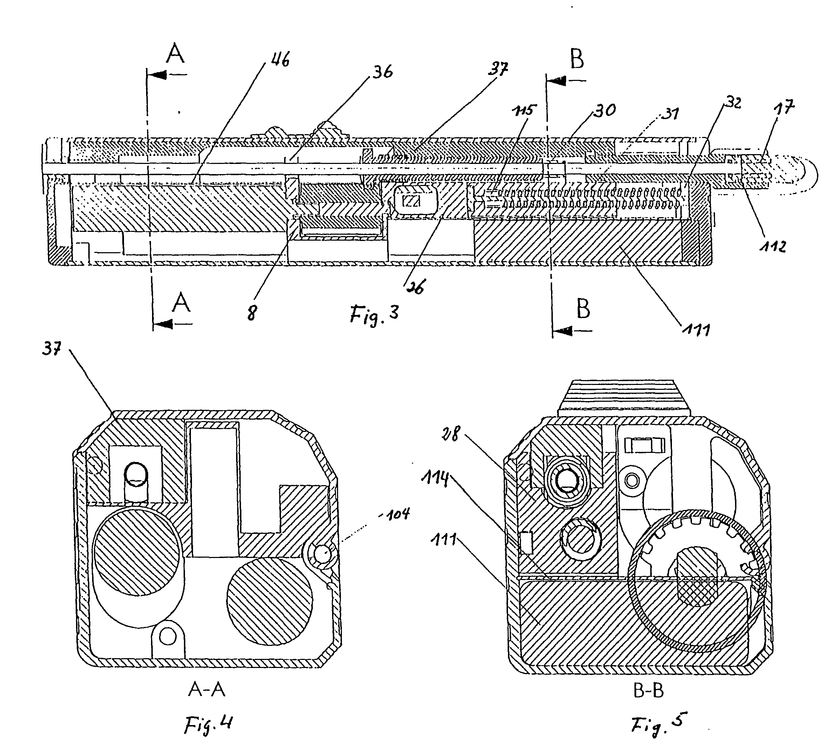

Fig. 13 zeigt den Antrieb und den Einbau der Vakuum-/Druckerzeugungsvorrichtung 5 (Blick von hinten, also entgegen der Z-Achse, Gehäusedeckel und Gehäuseunterteil sind weggelassen).
Im oberen, hinteren, rechten Bereich ist die Vakuum-/Druckerzeugungsvorrichtung 5 als Kolben-/Zylindereinheit 69 angeordnet. Sie besteht aus einem Spritzenkörper 52 mit darin angeordneter Gewindespindel 53, an deren dem Spritzenboden 51 zugewandten Ende ein Kolben 54 mit Dichtelementen - wie bei Spritzen allgemein bekannt - befestigt ist (Fig.14a - 14d).
An dem dem Basisblock 8 zugewandten Ende des Spritzenkörpers 52 ist auf der Gewindespindel eine Gewindespindelmutter 48 mit am Umfang ausgebildetem Zahnrad 55 angeordnet. Die Gewindespindelmutter hat eine oder mehrere Gewindegänge. Die Gewindespindel 53 wirkt mit der Gewindespindelmutter 48 zusammen. Die Spindel weist eine Steigung von ca. 5 mm pro Gang auf, so dass bei jeder Umdrehung der Kolben mittels des Spindelantriebs um einen genau definierten Betrag aus dem Spritzenkörper heraus, also vom Spritzenboden 51 weg, oder zum Spritzenboden hin, je nach Drehrichtung, bewegt wird. Der am Umfang der Gewindespindelmutter angeordnete Zahnkranz 55 kämmt mit dem Antriebsritzel 56, das auf der Abtriebswelle des Gleichstromgetriebemotors 58, befestigt ist. Die Abtriebswelle des Gleichstromgetriebemotors 58 ist im Basisblock 8 gelagert; hierfür ist die Abtriebswelle in die Querplatte 59 des Basisblocks eingesteckt. Wird der Gleichstromgetriebemotor 58 aktiviert, so wird der Kolben je nach Drehrichtung zum Spritzenboden oder in Richtung Basisblock 8 hin bewegt. Als Antriebsmotor wird ebenfalls ein Gleichstrommotor mit hoher Drehzahl verwendet, dem ein Planetengetriebe mit hoher Untersetzung nachgeschaltet ist. Er entspricht dem bereits beschriebenen Motor für die Spanneinrichtung.
Der Kolben 54 ist in bekannter Weise als Spritzenkolben ausgebildet. Der aus Kunststoff gefertigte Spritzenkörper, ein Zylinder mit Boden, ist durchsichtig.
Um ein Verdrehen der Gewindespindel 53 beim Antrieb der Gewindespindelmutter zu verhindern, sind die beiden gegenüber liegenden Flächen 60 der Gewindespindel flächig ausgebildet (Fig. 14d). Die Gewindespindel wird mit dem freien Ende in das Einlegeelement eingelegt. Der Abstand der Flächen der Gewindespindel entspricht der Breite des U-förmigen Einlegeelements 62 des Basisblocks 8. Zwischen U-förmigem Querschnitt des Einlegeelements und den beidseitigen Spindelflächen besteht nur ein geringes Spiel. Die Gewindespindelmutter stützt sich am Basisblock ab.
Um ein Herausgleiten des Spritzenkörpers 52 beim Verdrehen der Gewindespindelmutter zu verhindern, ist die Anlagefläche am Basisblock 8 leicht konisch nach unten ausgebildet.
Der Stutzen 63 des Spritzenkörpers 52 ist in die Durchführung 16 des Gehäuseenddeckels 7 so eingelegt, dass der Spritzenkörper in etwa in waagrechter Lage gehalten wird.
Um das Verdrehen der Gewindespindel leichtgängig zu machen, weist die Gewindespindelmutter mit Zahnkranz auf der dem Basisblock zugewandten Seite eine etwa 1,5 mm starke Anphasung 66 auf. Da darüber hinaus die Fläche der Rippe 59 am Basisblock 8, die mit der Anphasung 66 der Gewindespindelmutter 48 zusammenwirkt, von oben nach unten geneigt ist, wird die Vakuum-/Druckerzeugungsvorrichtung bei Betrieb nach unten gezogen. Zur Erzeugung eines ausreichenden Vakuums von ca. 200 hph im Probeentnahmeraum wird z.B. bei einer Biopsienadellänge von ca. 250 mm und einem Innendurchmesser der Biopsiehohinadel von ca. 5 mm ein Spritzenkörper für 20 ml mit einer Länge von ca. 90 mm verwendet. Um den Spritzenkörper auch als Druckerzeuger verwenden zu können ist nach etwa ¾ der Länge, entsprechend dem Hub für das Erzeugen des Vakuums (Stellung gemäß Fig. 11b) eine Belüftungöffnung 67 von z.B. ca. 1,5 mm Durchmesser vorgesehen. Wird der Spritzenkolben über die Belüftungsöffnung 67 hinaus bewegt (Fig. 14c), - wenn das Vakuum nicht mehr benötigt wird - wird durch Luftzufuhr (Atmosphärendruck) über die Belüftungsbohrung 67 das vorher aufgebaute Vakuum in der Biopsiehohlnadel abgebaut. Wird danach die Drehrichtung des Getriebemotors umgekehrt, so wird durch Hineinfahren des Kolbens (zum Spritzenboden hin) die Vakuum-/Druckerzeugungsvorrichtung im System einen Überdruck aufbauen, was nach Öffnen des Probeentnahmeraums den Auswurf der Gewebeprobe bewirkt. Im Übrigen wird durch die Druckluft nicht nur der Probeentnahmeraum, sondern insbesondere auch der Innenraum der Biopsienadel gereinigt. Durch den den Nadelhohlraum einengenden Stopfen wird das Eindringen von Gewebeteilen in den Biopsienadelhohlraum erschwert, bzw. ganz verhindert. Durch die Einengung des Nadelhohlraums durch den Stopfen 79 wird der Druck am Probeentnahmeraum erhöht und dadurch der Auswurf der Probe gerade bei halb geöffnetem Probeentnahmeraum verbessert.
Nachfolgend wird die Bedienung der Biopsieeinrichtung näher erläutert:
Das herausnehmbare Einlegeelement 20, bestehend aus Vakuum/Druckerzeugungsvorrichtung, elastischem Verbindungselement sowie Biopsienadelträger mit Nadel und Schneidhülse und weiteren damit verbundenen Elementen, sowie eine auf die Nadel aufgesetzte Führungsrolle 81. Diese Einheit einschließlich einer Einlegehilfe wird steril verpackt angeliefert. Der Kolben 54 im Spritzenkörper 52 ist bei Lieferung geringfügig (1-2 mm) vom Spritzenboden abgehoben, der Probeentnahmeraum 71 der Biopsienadel 2 ist geöffnet um so vor dem Einlegen eine visuelle Überprüfung des Probeentnahmeraums vornehmen zu können. Nach dem Öffnen des Gehäusedeckels 10 wird das Trägerelement 37, einschließlich Biopsienadel 2, Schneideinrichtung 3 und anderer damit verbundener Teile, wie die am Verbindungselement 4 angeschlossene Vakuum-/Druckerzeugungsvorrichtung 5 in die hierfür vorgesehenen Verbindungselemente eingelegt (Fig. 2). Beim Einlegevorgang ist darauf zu achten, dass das Zahnrad 74 in die Zähne der Zahnwalze 23 eingreift; die Schneidhülse wird von oben in die U-förmige Halterung 36 eingelegt, gleichzeitig werden die Laschen 40 des Spannschlittens in die Ausnehmungen 77 des Trägerelements eingeführt; die Führungsrolle 81 wird in die Durchführung 13 eingelegt, so dass die Flanken 101 und 102 den Gehäuseenddeckel 6 umfassen. Die Schneidhülse ist in der Führungsrolle längs verschieblich und frei drehbar gelagert; die Führungsrolle selbst ist jedoch gegenüber der Schneidhülse nach dem Einlegen in den Gehäuseenddeckel nicht mehr verschieblich. Die Vakuum/Druckerzeugungsvorrichtung wird anschließend einerseits in das nach oben offene Einlegeelement 62 des Basisblocks 8 mit dem freien Ende 61 und andererseits in die U-förmige, nach oben offene Durchführung 16 mit dem Stutzen 63 eingelegt. Der Stutzen 63 liegt oberhalb des Schaltstifts 19. Da das basisblockseitige Einlegeelement eine lichte Breite aufweist, die gerade das Einlegen der beidseitig mit Flächen 60 versehenen Gewindespindel zulässt, ist die Gewindespindel im Einlegeelement drehsicher gehalten. Der Zahnkranz 55 der Gewindespindelmutter 48 greift nach dem Einlegen in das Abtriebsritzel 56 des Getriebemotors ein. Der Abstand zwischen dem Basisblock einerseits und dem Gehäuseenddeckel 7 andererseits ist so gehalten, dass der Spritzenkörper 52 mit auf dem Spritzenkörper aufgesetzter Gewindespindelmutter 48 gerade Platz findet. Die Einheit Spritzenkörper und aufgesetztes Zahnrad ist dadurch so gehalten, dass sie nicht axial verschiebbar ist. Nach dem Einlegen liegt die Vakuum/Druckerzeugungsvorrichtung parallel zum Biopsienadelträger; das Verbindungselement beschreibt einen Bogen von ca. 180 °.
Nachzutragen wäre noch, dass das Einlegen bei nicht gespanntem Spannschlitten erfolgt; dies bedeutet, dass das Zahnrad 74 bei geöffnetem Probeentnahmeraum am rechten Ende der Zahnwalze eingreift (Fig. 3). Nach dem korrekten Einlegen kann der Gehäusedeckel geschlossen werden.
Um den Einlegevorgang zu erleichtern, kann eine Einlegehilfe benutzt werden. Beim Schließen des Gehäusedeckels wird der Stutzen 63 nach unten gedrückt und dabei über den im Gehäuseenddeckel eingebauten Schaltstift 19, der Mikroschalter betätigt. Dadurch wird das elektrische System aktiviert, was durch Blinken der Resetdiode 91 an der Vorderfront des Handstückes angezeigt wird. Die Resetdiode blinkt zunächst grün, was bedeutet, dass die Positionierung der einzelnen Elemente, d.h. der Einlegevorgang, noch nicht beendet ist; der Gleichstromgetriebemotor 21 muss zunächst den Probeentnahmeraum 71 mit der Schneidhülse 3 verschließen (der Probeentnahmeraum war beim Einlegen teilweise geöffnet). Dies geschieht, durch Verdrehen der mit der Schneidhülse verbundenen Gewindehülse. Die Schneidhülse wandert nach links bis das Zahnrad 74 an der Innenseite der Halterung 36 zur Anlage kommt. Die Kunststoffscheibe 78 liegt nach dem Schließen des Probeentnahmeraums an der Halterung 36 (Innenseite) an. Der Gleichstromgetriebemotor 58 bringt während dieses Vorgangs oder vorher oder danach den Spritzenkolben 54 in Anlage mit dem Spritzenboden 51. Nachdem die Ausgangspositionen für die Vakuum/Druckerzeugungsvorrichtung und die Biopsienadel/Schneidhülse, erreicht sind, leuchten die Spanndiode 94 und die Probeentnahmediode 92 grün auf, die Resetdiode erlischt . Der Bediener muss sich jetzt entscheiden, ob er das Spannen des Spannschlittens einleiten, oder eine weitere Probe entnehmen will, z.B. weil er vorher bereits eine Gewebeprobe entnommen hat. Drückt der Bediener die Spanntaste 90, so wird das Spannen des Spannschlittens eingeleitet; die Spanndiode blinkt grün, die Probeentnahmediode 92 erlischt. Durch Drücken der Spanntaste erhält der elektrische Gleichstromgetriebemotor 21 Strom und der Gleichstromgetriebemotor treibt die Zahnwalze 23 an. Das mit der Zahnwalze 23 kämmende Zahnrad 74 dreht die Spindelwelle und gleichzeitig die damit verbundene Schneidhülse 3. Da die Spindelmutter 75 im Biopsienadelträger 37 eingepresst ist und das Zahnrad 74 sich über die Kunststoffscheibe 78 an der Halterung 36 abstützt, die über den Basisblock 8 mit dem Gehäuse fest verbunden ist, bewirkt das Drehen der Gewindespindelhülse 73, dass der Biopsienadelträger nach rechts bewegt wird. Gleichzeitig wird die mit dem Biopsienadelträger über das Lagerelement 49 verbundene Biopsienadel 2 mitgenommen, was dazu führt, dass die Biopsienadelspitze in die Schneidhülse hinein wandert. Der Biopsienadelträger 37 wird über die Ausnehmung/Laschenverbindung des Spannschlittens gegen die Wirkung der Spiralfeder 31 nach rechts verschoben bis der Hebel 33 des Verrastelementes in die Ausnehmung 82 des Spannschlittens durch die Feder 34 eingedrückt wird. Der Spannschlitten ist in dieser Position verriegelt. Der Getriebemotor erhält den Steuerbefehl dass die Verriegelungsposition erreicht ist, z.B. über eine in die Gleitfläche der Abdeckplatte eingelassene Fotozelle, die mit dem zurückgefahrenen Biopsienadelträger zusammenwirkt; die Drehrichtung des Motors wird umgekehrt und die Schneidhülse wird um den Betrag nach rechts zurückgedreht, um den die Schneidhülse durch Verschieben des Spannschlittens und die Biopsienadel über die Biopsienadelspitze hinaus gewandert war. Am Ende dieses Schrittes verschließt die Schneidhülse den Probeentnahmeraum vollständig (Fig. 11d), wie zu Beginn des Spannvorgangs. Die Verriegelungsdiode 95 leuchtet grün; das Blinken der Spanndiode 94 erlischt. Damit beim Spannvorgang die Reibkraft zwischen Zahnrad und Abstützelement verringert wird, ist die Kunststoffscheibe 78 zwischen Zahnrad 74 und Halterung 36 angeordnet. Nun wird die Biopsienadel der Biopsieeinrichtung z.B. in eine vorher gesetzte Koaxialkanüle eingesetzt. Das proximale Ende der gesetzten Koaxialkanüle enthält eine Dichtung, die so bemessen ist, dass sie den Raum zwischen Schneidhülse und Kanüle einerseits abdichtet, andererseits ein leichtes Einschieben der Biopsienadel mit Schneidhülse zuläßt. Durch den Dichtring wird verhindert, dass Luft von außen über den Raum zwischen Kanüle und Schneidhülse eingesaugt wird. Ebenso verhindert der Dichtring ein Austreten von Flüssigkeit (zytologischem Material) nach Einführung bzw. Einschießen der Biopsienadel. So wird die Möglichkeit einer Verschmutzung des desinfizierten Handstücks nahezu vermieden; andererseits verhindert die Flanke 101 der sterilen Führungsrolle 81, dass von Seiten des Handstücks eine Verschmutzung der sterilen Kanüle stattfindet. Die Biopsienadelspitze wird in der Kanüle an die Geschwulst herangeführt und nach korrekter Positionierung in die Geschwulst eingeschossen.
Der Schuss wird durch Drücken der Betätigungstaste 88 ausgelöst. Das Drücken hat zur Folge, dass durch Verschwenken des doppelarmigen Hebels 33 um die Achse 35 der Spannschlitten freigegeben wird . Der Spannschlitten wird durch Federwirkung nach links geschleudert. Die Probeentnahmediode leuchtet grün auf, die Spanndiode erlischt. Durch Betätigung der Programmtaste 89 wird der Ablauf für die Probeentnahme freigegeben; die Probeentnahmediode 92 blinkt grün. Zunächst wird der Gleichstromgetriebemotor 58 der Vakuum-/Druckerzeugungsvorrichtung aktiviert. Der Kolben der Vakuum-/Druckerzeugungsvorrichtung wird in Richtung Basisblock , also vom Spritzenboden weg bewegt, bis er eine Stellung kurz vor Freigabe der Belüftungsbohrung 67 erreicht (Fig. 14b). Das Vakuum im System ist erzeugt. Nach Erreichen seiner Endstellung aktiviert das System den Motor 21, die Schneidehülse, die den Probeentnahmeraum verschließt, wird über den Zahnrad-/Spindelantrieb geöffnet. Während des Öffnungsvorganges wird durch den im System herrschenden Unterdruck nacheinander das Gewebe und eventuelle zytologische Flüssigkeit (zytologisches Material) in den Probeentnahmeraum eingesaugt. Zytologische Flüssigkeit wird auch durch das Vakuum in den Biopsienadelhohlraum und in die Vakuum/Druckerzeugungsvorrichtung fließen. Dabei hat es sich als vorteilhaft erwiesen, dass durch den Stopfen (79) der Unterdruck vor allem auf den unteren Bereich, die untere Seite, des Probeentnahmeraums gelenkt wird und durch den Stopfen 79 ein Eindringen des Gewebes in die Biopsiehohlnadel erschwert, bzw. verhindert wird. Ist der Probeentnahmeraum vollständig geöffnet - die Gewebeprobe ist in den Probeentnahmeraum eingelagert - , wird der Getriebemotor 21 umgesteuert und der Probeentnahmeraum 39 geschlossen. Durch Drehen der Schneidhülse wird durch die Schneidkante 72 der Schneidhülse 3 beim Schließvorgang das Gewebe abgetrennt.

Um das Gewebe, die Gewebefäden, sicher zu durchtrennen, ist es vorteilhaft, die Schneidhülse 3 über das distale Ende des Probeentnahmeraums hinaus (ca. 2mm) zu verschieben. Um dies zu bewirken ist lediglich der Mikroprozessor, in dem diese Steuerdaten abgelegt sind, entsprechend zu programmieren. Durch die spezielle Gestaltung des Probeentnahmeraums und infolge des angelegten Vakuums ist die Gewebeprobe drehgesichert im Probeentnahmeraum gehalten, so dass durch die die Biopsienadel außen umgebende, drehend längsverschiebliche Schneidhülse 3 die Gewebeprobe, wie beschrieben, nicht verdreht oder verdrillt wird. Nachdem der Probeentnahmeraum geschlossen ist, wird der Gleichstromgetriebemotor für die Vakuumerzeugungseinheit 5 aktiviert. Der Kolben 54 wird zunächst soweit zurückgefahren bis der Kolben die Belüftungsöffnung freigibt (Fig. 14c). Nach Abbau des Vakuums im System fährt der Kolben soweit zum Spritzenboden vor, dass die Belüftungsbohrung wieder verschlossen wird, um den Ausfluss von Körperflüssigkeit (zytologische Flüssigkeit) zu verhindern. Das Blinken der Probeentnahmediode 92 erlischt. Die Auswurfdiode 93 leuchtet grün. Die Biopsienadel mit geschlossenem Proberaum wird aus der Kanüle gezogen. Nach der Entnahme der Biopsieeinheit und Bereitstellung eines Gefäßes für die Aufnahme der Gewebeprobe und Flüssigkeit, wird die Programmtaste 89 erneut betätigt und die Auswurfdiode 93 beginnt zu blinken. Zunächst wird der Getriebemotor 21 der Schneidhülse betätigt um den Probeentnahmeraum etwa bis zur Hälfte zu öffnen. Danach wird der Gleichstromgetriebemotor 58 der Vakuum-/Druckerzeugungsvorrichtung aktiviert. Die Drehrichtung des Gleichstromgetriebemotors 58 bleibt und die Gewindespindel 53 mit Kolben bewegt sich in Richtung Spritzenboden, so dass im System nun ein Überdruck entsteht. Der Kolben wird bis zum Spritzenboden vorgefahren, der Antriebsmotor 58 wird deaktiviert. Der Getriebemotor 21 fährt die Schneidhülse über dem Probeentnahmeraum weiter zurück, nachdem der Kolben den Kolbenboden erreicht hat. Infolge des im System aufgebauten Überdrucks wird die Probe unter Druck schon bei halb geöffnetem Probeentnahmeraum in ein bereitstehendes Laborgefäß herausgedrückt, gleichzeitig wird der Hohlraum Vakuum-/Druckerzeugungsvorrichtung, der Biopsienadel und der Probeentnahmeraum von Gewebepartikeln und Flüssigkeit befreit. Der Auswurf der Probe bei etwa halb geöffnetem Probeentnahmeraum erfolgt deshalb, weil dadurch der Auswurf der Gewebeprobe sichergestellt wird und nicht durch vorzeitigen Abbau des Überdrucks die Gewebeprobe in den Probeentnahmeraum zurückfällt. Die Einengung des Biopsienadelhohlraums durch den Stopfen 79, der ein Eindringen von Gewebe in den Biopsienadelhohlraum erschwert, bzw. verhindert hat, erweist sich bei der Probeentnahme als besonders vorteilhaft, da der verengte Querschnitt den Auswurfdruck erhöht. Die besten Auswurfergebnisse wurden deshalb bei halb geöffnetem Probeentnahmeraum erzielt; d.h. die Schneidhülse gibt die Hälfte des Probeentnahmeraums frei. Durch den Überdruck wird auch die Gewebeflüssigkeit aus dem Probeentnahmeraum gedrückt und dieser gereinigt.
Nachdem der Probeentnahmeraum vollständig geöffnet ist, ist die Entnahme und Reinigung abgeschlossen, die Auswurfdiode erlischt. Die Resetdiode 91 leuchtet grün. Sofern nun keine weitere Probe entnommen werden soll, wird der Gehäusedeckel geöffnet und das herausnehmbare Element 20 entfernt. Beim Öffnen des Gehäusedeckels 10 wird das System über den Mikroschalter 18 deaktiviert. Soll jedoch eine weitere Probe aus der gleichen Gewebeumgebung entnommen werden, so drückt der Bediener die Programmtaste 89 und die Resetdiode 91 beginnt zu blinken. Die Einstellung der Vakuum-/Druckerzeugungsvorrichtung, wie der Schneidhülse findet, wie beschrieben, erneut statt. Nach Abschluss des Vorgangs erlischt die Resetdiode 91 und die Probeentnahmediode leuchtet auf. Die folgenden Prozessschritte laufen in der bereits beschriebenen Reihenfolge ab. Der Vorgang kann beliebig oft wiederholt werden. Der Bediener muss nach Abschluss lediglich entscheiden, ob er eine weitere Probe entnehmen will oder die Probeentnahme abgeschlossen ist und der Gehäusedeckel geöffnet wird.
Wie bereits beschrieben, kann zur Erhöhung der Bediensicherheit für einzelne Schritte, z.B. "Spannen" und "Auswurf der Probe" eine Verzögerungsschaltung vorgesehen sein. Weiterhin können die Leuchtdioden unterschiedliche Farben haben, so dass zwischen Arbeiten im Gewebe und außerhalb des Gewebes unterschieden wird.
Sollte es erforderlich sein, dass die Probe an einer Stelle der Geschwulst entnommen wird, die nach dem Einschuss nicht direkt über oder am Probeentnahmeraum liegt, also z.B. seitlich davon, so kann die Lage des Probeentnahmeraumes 71 über die Rändelschraube 80 verdreht werden. Damit der Bediener diese radiale Stellung des Probeentnahmeraums erkennen kann, ist auf der Rändelscheibe eine Markierung in Form einer Kerbe 119 angebracht, die nach oben zeigt, wenn die Öffnung des Probeentnahmeraums nach oben zeigt. In der jeweils eingestellten Position wird die Biopsienadel durch die Flächen des Vielkants 50 und die elastischen Kräfte im Trägerteil fixiert. Der Vorgang der Probeentnahme ist der gleiche wie bereits beschrieben.
Nach Beendigung der Biopsie wird nach Entrastung des Deckels das austauschbare Element 20 (Vakuum-/Druckvorrichtung, Biopsienadel/Schneidvorrichtung mit allen daran angeordneten Elementen) nach oben entnommen. Um ein Öffnen des Gehäuses bei gespanntem Spannschlitten unmöglich zu machen, ist an den Biopsienadelträger ein Sicherungsflügel 84 angeordnet, der im gespannten Zustand an der linken Stirnfläche 85 der Verschlusseinrichtung anliegt. Die in der X-Achse verschiebbare Verschlusseinrichtung lässt sich dadurch nicht mehr nach links in Öffnungsstellung bewegen und damit kann die Nase 12 nicht mehr aus der Ausnehmung 45 heraus genommen werden. Umgekehrt lässt sich auch der Gehäusedeckel nicht schließen, sofern die Trägereinheit bei gespanntem Zustand eingelegt wurde, da der Sicherungsflügel verhindert, dass der Riegel in den dafür vorgesehenen Raum eingefügt werden kann. Die Fläche 85 des Riegels stößt am Sicherungsflügel an. Die Batterieladediode 96 ist abgeschaltet, sobald der Gehäusedeckel geöffnet ist. Bei geschlossenem Deckel und eingelegtem Einlegeelement 20 zeigt die Batterieladediode an, ob ausreichend Energie vorhanden ist.

Grundsätzlich ist es denkbar, dass alle Schritte für die Entnahme einer Probe sowie das Spannen des Schlittens usw. durch Aktivierung und Deaktivierung der beiden Getriebemotoren einzeln von Hand gesteuert werden. Es ist aber zweckmäßig, dass einzelne Schritte des Ablaufvorgangs zusammengefasst werden und automatisch ablaufen und nur die Einleitung des Folgeschritts durch Schalterbetätigung in Gang gesetzt wird. Diese halbautomatische Methode, wie vorher beschrieben, hat sich als besonders vorteilhaft erwiesen.
Grundsätzlich sind zwei Methoden zur Erfassung der Ist-Werte für den Vergleich mit den Soll-Werten denkbar. Die eine Methode beruht auf dem Messen der Längenverschiebung der Gewindespindel beim Herausziehen, bzw. Hineindrücken sowie dem Messen der axialen Verschiebung der Schneidhülse, bzw. des Biopsienadelträgers. Um diese Veränderungen zu erfassen sind Fotozellen oder Mikroschalter im Gehäuseinneren, insbesondere auf der Verlängerung des Basisblocks 8, angeordnet. Auf der Schneidhülse ist zusätzlich ein Positionierungsfinger 103 aufgesetzt, während bei der Gewindespindel der Vakuum-/Druckerzeugungsvorrichtung das aus der Kolbeneinheit herausragende freie Ende 61 als Messpunkt herangezogen werden kann. sofern die vordere Kante des Biopsienadelträgers als Messpunkt mit einer Fotozelle verwendet wird, bedarf es keines zusätzlichen Positionierungsfingers. Die eingelassenen Fotozellen werden wegen einer eventuellen Verschmutzung mit geeignetem, durchsichtigen Material abgedeckt. Der Positionierungsfinger 103 durchgreift einen Schlitz im Biopsienadelhalter. An entsprechenden Stellen, an der Verlängerung 46 des Basisblocks 8 sind Ausnehmungen 107 vorgesehen, in die Fotozellen, bzw. Mikroschalter eingebaut sind, die entweder mit dem freien Ende 61 der Kolbenspindel, mit dem Positionsfinger oder der Biopsienadelträgerkante 120 zusammenwirken (Fig. 15). Diese Signale (Ist-Wert) werden in der Elektronik verarbeitet und bilden die Steuersignale.

Das andere System beruht auf der Messung der Umdrehungszahl der Gleichstrommotoren. Hierbei wird auf die Welle des Gleichstrommotors ein Geber aufgesetzt, der mit einer auf dem Gehäuse des Gleichstrommotors aufgesetzten Fotozelle zusammenwirkt. Dadurch wird die Umdrehungszahl des Motors gemessen. Da die Gleichstrommotoren lastabhängig mit einer Drehzahl von ca. 10.000 - 12.000 U/min. arbeiten und andererseits das nachgeschaltete, abtriebsseitig angeordnete Planetengetriebe, das mit dem Spindelantrieb zusammenwirkt, die Umdrehungszahl erheblich reduziert, ist eine genaue Längssteuerung möglich. Die Längsverschiebung durch den Spindelantrieb ist proportional zur Abtriebsumdrehung ein stets gleicher Betrag und ist daher als Steuersignal für die Längsverschiebung ausreichend. Um die Position der Schneidhülse 3 sowie des Kolbens 54 bei Beginn, also nach dem Einlegen des herausnehmbaren Elements und Schließen des Gehäusedeckels 10 genau zu bestimmen, dreht der Gleichstromgetriebemotor 58 den Kolben 54 auf Anschlag auf den Spritzenboden und der Gleichstromgetriebemotor 21 bringt den Schneidhülsenantrieb auf Null-Stellung, indem er das Zahnrad 74 an der Gewindespindelmutter 75 zum Anschlag bringt (Die Gewindespindelmutter 75 läuft auf das Zahnrad 74 auf). Von dieser Null-Position aus wird dann über den Vorabe-Ist-Vergleich die Steuerung der einzelnen Schritte durchgeführt. Die erforderlichen Kabel vom Messgeber zur Elektronik sind im Gehäuse untergebracht, ebenso die Platine mit den elektronischen Bauteilen.

Ein im Inneren des Gehäuses unter der Abdeckung angeordneter Mikroprozessor, in dem die Vorgaben abgelegt sind, steuert die einzelnen Vorgänge.
Um die austauschbare Einlegeeinheit leicht einsetzen zu können, kann die in Fig. 16, 17) gezeigte Einlegehilfe verwendet werden. Wie insbesondere Fig. 16, 17 zeigen, wird der Biopsienadelträger von zwei Laschen 108 umfasst und durch einen zusätzlichen Steg 109 in der Halterung axial fixiert, so dass er parallel zur Vakuum-/Druckvorrichtung in der Einlegehilfe zu liegen kommt. Die Vakuum/Druckerzeugungsvorrichtung wird ebenfalls einerseits von der Lasche 116 und andererseits von der mittig angeordneten Lasche 108 umfasst. Zusätzlich greift ein Stift 110 in die Belüftungsbohrung 67 ein. Dadurch ist gewährleistet, dass die Vakuum/Druckerzeugungsvorrichtung parallel zum Biopsienadelträger ausgerichtet ist (Fig.1). Die so ausgerichteten Teile sind so in der Einlegehilfe fixiert, dass sie mittels der Haltestücke 117 leicht von oben her in das Handstück einlegbar sind. Da die Teile mit Einlegehilfe steril verpackt sind, kann das Auswechselelement 20 ohne Handberührung der Verpackung entnommen und steril in das Handstück 1 eingesetzt werden. Zur leichteren Aufnahme der Vakuum-/Druckerzeugungsvorrichtung und des Biopsienadelträgers sind die Laschen leicht abgeschrägt. Da die Einlegehilfe aus Kunststoff gefertigt ist, können durch entsprechende Wahl der Toleranz und Flexibilität die aufzunehmenden Teile leicht durch Klemmung gehalten werden.

Die Spitze der Nadeleinheit der Biopsievorrichtung kann an das zu biopsierende Gewebe direkt angesetzt werden und in das Gewebe eingeschossen werden. Es kann aber zweckmäßig sein, vor dem Einsatz des Gerätes eine Koaxialkanüle zu positionieren und anschließend den Teil der Nadeleinheit (bestehend aus Biopsienadel und Schneidhülse), der aus dem Handstück der Biopsievorrichtung herausragt, in die Koaxialkanüle 125 einzuführen. Hierbei ist darauf zu achten, dass zwischen Innenfläche der Koaxialkanüle und Außenfläche der Nadeleinheit beim Aufbau des Vakuums das für das Einsaugen der Gewebeprobe nötig ist, keine Luft von außen in diesen Raum eindringen kann. Bei der aus einem Rohr 121 mit proximalseitig aufgesetzter Kappe 122 bestehende Koaxialkanüle (Fig. 18) weist das Rohr 121 am proximalen Ende ein Dichtelement 123 auf (z.B. einen entsprechend dimensionierten Silikonschlauch), in das die Nadeleinheit eingesetzt wird. Zum Einsetzen der Koaxialkanüle wird ein Dorn 124 mit der Koaxialkanüle 125 verbunden. Der Dorn 124 weist eine Spitze 126 auf, die über das distale Ende der Koaxialkanüle im eingesetzten Zustand hinausragt. Die Verbindung von Koaxialkanüle und Dorn erfolgt z.B. über einen Schraubverschluss, weshalb die Dornkappe als Schraubkappe ausgebildet ist. Die Schraubkappe wird auf das proximale Ende der Kappe 122 aufgeschraubt. Das Rohr der Koaxialkanüle wird in der Kappe 122 z.B. durch Klemmsitz gehalten. Nach dem einsetzen der Koaxialkanüle wird der Dorn entfernt und die Nadeleinheit der Biopsievorrichtung (im gespannten Zustand) in die Koaxialkanüle eingebracht und positioniert (Fig. 20) Dabei sitzt die distale Flanke der Führungsrolle 101 auf der proximalen Stirnfläche 128 der Kappe auf. Nach dem Entriegeln des Spannschlittens dringt die Nadelspitze mit dem Probeentnahmeraum in voller Länge in das Gewebe ein.

Die Eindringtiefe der Biopsienadeleinheit der Biopsievorrichtung liegt je nach gewählter Nadelgröße zwischen 20 und 35 mm. Im Allgemeinen beträgt sie 20 mm. Bei kleinen Brüsten oder Geschwulsten, die knapp unter der Haut liegen, ist daher die Eindringtiefe der Biopsienadel beim Einschuss zu tief, da das Biopsiegerät auf der Koaxialkanüle unmittelbar oder mittels der Führungsrolle aufgesetzt wird und die Eindringtiefe nicht am Gerät veränderbar ist. Die Eindringtiefe ist gerätefest.
Um das gleiche Biopsiegerät mit gleicher Biopsienadel und gleicher Einschusstiefe und die gleiche, also eine einheitliche Koaxialkanüle mit gleicher Gesamtlänge bei geringeren Einschusstiefen verwenden zu können, wird auf die Biopsienadel vor dem Einsetzen ein oder mehrere Distanzstücke 129 medial aufgesetzt; sie liegen also medial vor der im Gehäuse gelagerten Führungsrolle 101 und der proximalen Stirnfläche 128 der Kappe 122. Durch das Einfügen von Distanzstücken oder einem Distanzstück kann somit die Eindringtiefe T bei gleicher im Gerät vorgesehener Einschusstiefe verändert werden. Nach dem Einsetzen des Distanzstückes steht die Nadelspitze der Biopsienadel im gespannten Zustand nicht mehr, wie beim Einsetzen ohne Distanzstück aus der Koaxialkanüle geringfügig vor, sondern sie liegt in der Koaxialkanüle. Die Eindringtiefe wird also um die Länge L des Distanzstückes reduziert (sh. auch Fig. 20 und 21). Die Funktion des Probeentnahmeraums 21 sowie die Betätigung der Schneidhülse wird dadurch nicht beeinträchtigt. Wird so z.B. ein Distanzstück von 10 mm bei einer Nadeleindringtiefe von 20 mm verwendet, so wird die Eindringtiefe auf 10 mm reduziert. Natürlich kann das Distanzstück einteilig oder mehrteilig ausgeführt werden, d.h. bei Verwendung von Distanzstücken mit 5 mm Stärke sind bei einer Reduktion der Eindringtiefe um 10 mm 2 Distanzstücke nötig.
Das Einfügen von Distanzstücken oder von einem Distanzstück entsprechender Länge bietet die Möglichkeit, eine einheitliche Koaxialkanüle einschließlich eines einheitlich einzufügenden Eindrückdorns 124 für verschiedene Eindringtiefen zu verwenden. Das gleiche Ergebnis bezüglich einer reduzierten Eindringtiefe könnte auch erzielt werden durch die Verwendung verschieden hoher Kappen oder durch Aufsetzen der Distanzstücke auf die Kappe, was ein Äquivalent zu den aufzufädelnden Distanzstücken darstellen würde.

### Teileliste

- 1: Handstück
- 2: Biopsienadel
- 3: Schneidhülse
- 4: Verbindungselement
- 5: Vakuum-/Druckerzeugungsvorrichtung
- 6: Gehäuseenddeckel (links)
- 7: Gehäuseenddeckel (rechts)
- 8: Basisblock
- 9: Gehäuseunterteil
- 10: Gehäusedeckel
- 11: Verschlussriegel
- 12: Nase
- 13: Durchführung
- 14: Bohrung
- 15: Durchführung
- 16: Durchführung
- 17: Zapfen
- 18: Mikroschalter
- 19: Schaltstift
- 20: herausnehmbares Element
- 21: Gleichstromgetriebebemotor
- 22: Wand
- 23: Zahnwalze
- 24: U-förmiger Raum
- 25: Wand
- 26: Block
- 27: Nut
- 28: Spannschlitten
- 29: Gewindebohrung
- 31: Spiralfeder
- 32: Endstück
- 33: Doppelhebel
- 34: Druckfeder
- 35: Achse
- 36: Halterung
- 37: Biopsienadelträger
- 38: Bohrungen
- 39: N.N.
- 40: Laschen
- 41: Fläche Spannschlitten
- 42: Verlängerung der Fläche
- 44: Fläche des Blocks 26
- 45: Ausnehmung
- 46: Abdeckung
- 47: Kunststoffteil
- 48: Gewindespindelmutter
- 49: Lagerelement
- 50: Vielkant
- 51: Spritzenboden
- 52: Spritzenkörper
- 53: Gewindespindel
- 54: Kolben
- 55: Zahnrad (Zahnkranz)
- 56: Antriebsritzel
- 57: Platine
- 58: Gleichstromgetriebemotor
- 59: Querplatte
- 60: Flächen
- 61: freies Ende
- 30: Bolzen
- 63: Stutzen
- 64: Ausflussstutzen
- 65: Aussparung
- 66: Anphasung
- 67: Belüftungsbohrung
- 68: N.N.
- 69: Kolben-/Zylindereinheit
- 70: Nadelspitze
- 71: Probeentnahmeraum
- 72: Schneide
- 73: Gewindespindelhülse
- 74: Zahnrad
- 75: Gewindespindelmutter
- 76: Dichtelement
- 77: Ausnehmungen
- 78: Kunststoffscheibe
- 79: Stopfen
- 80: Rändelscheibe
- 81: Führungsrolle
- 82: Ausnehmung
- 83: Metallteil
- 84: Sicherungsflügel
- 85: Stirnfläche
- 86: N.N.
- 87: Mittelrippe
- 88: Betätigungstaste
- 89: Programmtaste
- 90: Spanntaste
- 91: Resetdiode
- 92: Probeentnahmediode
- 93: Auswurfdiode
- 94: Spanndiode
- 62: Einlegeelement
- 96: Batterieladediode
- 97: Durchführung
- 98: Durchführung
- 99: Arm des doppelarmigen Hebels
- 100: Teil des Hebel
- 101: Flanken der Führungsrolle links
- 102: Flanken der Führungsrolle rechts
- 103: Positionsfinger
- 104: Achse
- 105: Antriebsvorrichtung (Vakuum)
- 106: Antriebsvorrichtung (Biopsienadel, Spanneinrichtung
- 107: Ausnehmungen Laschen
- 109: Steg
- 110: Stift
- 111: Akku
- 112: Kunststoffteil
- 113: Fläche
- 114: Trennplatte
- 115: Führungsbohrung
- 116: Befestigung
- 117: Haltestücke
- 118: N.N.
- 119: Kerbe
- 120: Nadelträgerkante
- 121: Rohr
- 122: Kappe
- 123: Dichtelement
- 124: Dorn
- 125: Koaxialkanüle
- 126: Spitze
- 95: Verriegelungsdiode
- 128: Stirnfläche
- 129: Distanzstück
- T =: Eindringtiefe
- L =: Länge des Distanzstückes
- 127: Schraubkappe

## Patentansprüche

1. Biopsievorrichtung zur Entnahme von Gewebeproben, bestehend aus einem Handstück, in das eine hohle Biopsienadel eingelegt wird, wobei ein Teil des über das Handstück hinausragenden Teils der Biopsienadel mit seinem Probeentnahmeraum in das zu untersuchende Gewebe eingebracht wird und das Gewebe mittels Vakuum in den Probeentnahmeraum eingesaugt und anschließend mittels einer Probeabtrenneinrichtung abgetrennt und anschließend entnommen wird, **dadurch gekennzeichnet, dass** der Spannschlitten mittels elektrischer Motorkraft gegen die Wirkung einer Feder in Spannstellung gebracht wird aur dem im Handstück gelagerten Spannschlitten die Nadeleinheit angeordnet ist und der Probeentnahmeraum in das Gewebe nach Entriegelung des vorgespannten Spannschlittens eingeschossen wird, die Vakuum-/Druckerzeugungsvorrichtung (5) sowie weitere Steuerungs- und Versorgungsvorrichtungen in das Gehäuse des Handstücks (1) integriert sind, und das Verbindungselement (4) von der Biopsienadel (3) zur Vakuum-/Druckerzeugungseinheit (5) unmittelbar am Gehäuse angeordnet ist, die Vakuum-/Druckerzeugungsvorrichtung (5) aus einer steuerbaren Kolben-/Zylindereinheit (69) besteht, die eine Belüftungsöffnung (67) aufweist, so dass in der Vakuum-/Druckerzeugungsvorrichtung zum Auswurf der Probe Überdruck erzeugt werden kann, dass alle Antriebe elektrisch betätigt sind und dass der Antrieb für den Spannschlitten auch als Antrieb für die Schneidhülse verwendet wird, dass die Biopsiehohlnadel von einer außen liegenden, koaxialen Schneidhülse umgeben ist und dass auf der Frontseite des Gehäuses eine Platine für die Betätigung der Elektronik angeordnet ist, in die der Entraster des Spannschlittens integriert ist.

2. Biopsievorrichtung nach Anspruch 1, **dadurch gekennzeichnet, dass** die Vakuum-Druckerzeugungsvorrichtung (5) eine Spritzen-/Kolbeneinheit ist, die im oberen Teil eine Belüftungsöffnung (67) aufweist, die zum Abbau des Vakuums durch Zurückziehen des Spritzenkolbens (54) geöffnet wird.

3. Biopsievorrichtung nach Anspruch 1 und 2, **dadurch gekennzeichnet, dass** der Kolben (54) der Vakuum-/Druckerzeugungsvorrichtung (5) mittels eines steuerbaren Spindelantriebs (53, 48) in beiden Richtungen bewegbar ist.

4. Biopsievorrichtung nach Anspruch 3, **dadurch gekennzeichnet, dass** als Antrieb für den steuerbaren Spindelantrieb (53, 48) ein hochtouriger Elektro-Gleichstrommotor (58) mit nachgeschaltetem Planetengetriebe verwendet wird.

5. Biopsievorrichtung nach Anspruch 4, **dadurch gekennzeichnet, dass** die Übertragung vom Planetengetriebe auf die Kolbenspindel (53) über ein einstufiges Zahngetriebe erfolgt, wobei die auf den Spritzenzylinder aufgesetzte Gewindespindelmutter (48) auf der Außenseite einen Zahnkranz 55 trägt.

6. Biopsievorrichtung nach Anspruch 1 - 4, **dadurch gekennzeichnet, dass** der Kolben (54) zum Erzeugen eines Vakuums im System und im Probeentnahmeraum in einem ersten Schritt vom Spritzenboden (52) weg bis kurz vor die Belüftungsöffnung (67) bewegt wird.

7. Biopsievorrichtung nach Anspruch 1 - 4, **dadurch gekennzeichnet, dass** zur Belüftung des Systems der Kolben (54) in einem dem ersten Schritt folgenden zweiten Schritt über die Belüftungsöffnung (67) hinaus zurückgezogen wird, und nach Abbau des Vakuums wieder zurückgefahren wird um die Belüftungsbohrung zu verschließen.

8. Biopsievorrichtung nach Anspruch 1 - 4, **dadurch gekennzeichnet, dass** der Kolben (54) in einem dem zweiten Schritt folgenden dritten Schritt in Richtung Spritzenboden (52) bewegt wird um im System und im Probeentnahmeraum einen Überdruck zu erzeugen.

9. Biopsieeinrichtung nach Anspruch 1, **dadurch gekennzeichnet, dass** der elektrische Getriebemotor (58) der Kolben-/Zylindereinheit (69) über eine Drehzahlmessung so gesteuert wird, dass der Kolben (54) in einem ersten Schritt aus dem Zylinder bis kurz vor die Belüftungsöffnung (67) zurückgezogen, in einem zweiten Schritt die Belüftungsbohrung (67) freigegeben wird und der Kolben nach Abbau des Vakuums die Belüftungsbohrung wieder verschließt, und in einem dritten Schritt in umgekehrter Richtung zur Erzeugung des Überdrucks zum Spritzenboden (51) hin in Abstimmung mit der Steuerung der Probeentnahme und dem Auswurf der Probe bewegt wird.

10. Biopsievorrichtung nach Anspruch 1 **dadurch gekennzeichnet, dass** die Umdrehungszahl der Gleichstrommotoren durch eine am Motorgehäuse fest angeordnete Fotozelle und einem auf der Motorwelle angeordneten Geber gemessen wird.

11. Biopsievorrichtung nach Anspruch 10, **dadurch gekennzeichnet, dass** die Umdrehungszahl des Motors mit einem vorher in der Elektronik gespeicherten Soll-Wert verglichen als Auslöser für die Steuerung des Spindelantriebs benutzt wird.

12. Biopsievorrichtung nach Anspruch 1, **dadurch gekennzeichnet, dass** der Innenraum der hohlen Biopsienadel (2) über ein Verbindungsstück (4) mit dem Innenraum der Vakuum-/Druckvorrichtung (5) so verbunden ist, dass bei Unterdruck keine Luft von außen einströmen oder bei Überdruck ausströmen kann.

13. Biopsievorrichtung nach Anspruch 12, **dadurch gekennzeichnet, dass** das Verbindungsstück (4) ein flexibler, in unmittelbarer Nähe des Handstücks (1) angeordneter Schlauch ist.

14. Biopsievorrichtung nach einem oder mehreren der vorhergehenden Ansprüche, **dadurch gekennzeichnet, dass** der Biopsienadelträger (37) mit der runden, hohlen Biopsienadel (2) und die koaxial die Biopsienadel umfassende ebenfalls querschnittsrunde Schneidhülse (3) sowie Teilen des Antriebs, dem Verbindungselement (4) und der Vakuum-/Druckerzeugungsvorrichtung (5) ein herausnehmbares, auswechselbares Element (20) bilden, das in das Handstück (1) einlegbar ist.

15. Biopsievorrichtung nach Anspruch 14, **dadurch gekennzeichnet, dass** der herausnehmbare Biopsienadelträger (37) mittels der Ausnehmungen (77) in die Laschen (40) des Spannschlittens (28) einsetzbar ist.

16. Biopsievorrichtung nach Anspruch 1, **dadurch gekennzeichnet, dass** der Spannschlitten (28) über einen mittels eines Gleichstromgetriebemotors (21) mit nachgeschaltetem einstufigen Getriebe (23, 74) angetriebenen Spindelantrieb (73, 75) in Spannstellung gebracht wird.

17. Biopsievorrichtung nach Anspruch 17, **dadurch gekennzeichnet, dass** der Spannschlitten (28) in der Spannstellung mechanisch verriegelbar ist.

18. Biopsievorrichtung nach einem oder mehreren der Ansprüche 14 - 17, **dadurch gekennzeichnet, dass** auf der Abtriebswelle des dem Gleichstromgetriebemotor nachgeschalteten Planetengetriebes eine Zahnwalze (23) sitzt, in die das Zahnrad (74) des mit der Schneidhülse (3) verbundenen Spindelantriebs (73, 75) eingreift.

19. Biopsievorrichtung nach Anspruch 18, **dadurch gekennzeichnet, dass** das auf dem Spindelantrieb (73), (75) aufgesetzte Zahnrad (74) sich bei Verschiebung des Spannschlittens (28) auf einer Halterung (36) des Basisblocks (8) abstützt.

20. Biopsievorrichtung nach Anspruch 1 und 14, **dadurch gekennzeichnet, dass** die Biopsienadel (2) mit koaxialer Schneidhülse (3) und weiteren daran angeordneten Elementen im Biopsienadelträger (37) an zwei Lagerstellen (75, 49) so gehalten sind, dass die Biopsienadel (2) und/oder die Schneidhülse (3) einzeln für sich verdrehbar sind.

21. Biopsievorrichtung nach Anspruch 1 und 14, **dadurch gekennzeichnet, dass** die Kolben-/Zylindereinheit (69) so ausgelegt ist, dass im System, bestehend vor allem aus der Hohlraum-Kolben-/Zylindereinheit (69), der hohlen Biopsienadel (2) und dem hohlen Verbindungsstück (4), im Probeentnahmeraum (71) ein erzeugtes Vakuum in der Größenordnung von 200hph angelegt wird.

22. Biopsievorrichtung nach Anspruch 1 und 14, **dadurch gekennzeichnet, dass** der Querschnitt der Biopsienadel vor dem Probeentnahmeraum eine Einengung (79) aufweist, die die Öffnung des Querschnitts des Probeentnahmebereichs von oben her überdeckt

23. Biopsievorrichtung nach Anspruch 22, **dadurch gekennzeichnet, dass** die Einengung 60 - 75% des Querschnittes umfasst und dass die Einengung von oben in den Querschnitt ragt.

24. Biopsievorrichtung nach Anspruch 23, **dadurch gekennzeichnet, dass** die Einengung ein ca. 10 mm langer Stopfen ist.

25. Biopsievorrichtung nach Anspruch 1 und 14, **dadurch gekennzeichnet, dass** die Einengung als eine in den Querschnitt hineinragende Lippe oder Nase ausgebildet ist.

26. Biopsievorrichtung nach Anspruch 1 und 14, **dadurch gekennzeichnet, dass** nur ca. 25% Querschnitts des Probeentnahmeraums (71) nach oben hin offen sind.

27. Biopsievorrichtung nach Anspruch 20, **dadurch gekennzeichnet, dass** die Gewindespindelmutter (75) der Gewindespindelhülse (73) in den Biopsienadelträger (37) eingepresst ist und eine der beiden Lagerstellen bildet.

28. Biopsievorrichtung nach Anspruch 1, **dadurch gekennzeichnet, dass** im Zentrum des Innengehäuses ein Basisblock (8) angeordnet ist, der sowohl zur Befestigung, als auch zur Abstützung, Lagerung und Halterung der einzelnen Bauteile wie Spannschlitten (28), Biopsienadelträger (37), Vakuum/Druckerzeugungsvorrichtung (3) und Antriebsvorrichtungen (105,106) dient.

29. Biopsievorrichtung nach Anspruch 1, **dadurch gekennzeichnet, dass** in dem für die gehäuseseitige Lagerung der Vakuum-/Druckvorrichtung (5) dienenden Gehäuseenddeckel (7) ein Mikroschalter (18) integriert ist, bei dessen Betätigung die Energieversorgung freigegeben wird.

30. Biopsievorrichtung nach Anspruch 29, **dadurch gekennzeichnet, dass** der Schaltstift (19) des Mikroschalters (18) durch Niederdrücken der Vakuum-/Druckerzeugungsvorrichtung (5) mittels des Gehäusedeckels (10) betätigt wird.

31. Biopsievorrichtung nach Anspruch 1 und 14, **dadurch gekennzeichnet, dass** am Biopsienadelträger (37) Mittel vorgesehen sind, die bei gespanntem Spannschlitten und eingelegtem Biopsienadelträger ein Schließen des Gehäuseeckels (10), bzw. ein Öffnen des Gehäusedeckels bei geschlossenem Gehäuse verhindern.

32. Biopsievorrichtung nach Anspruch 1, **dadurch gekennzeichnet, dass** am Gehäuse Flächen für die Befestigung des Handstückes an einer Positionierungseinrichtung vorgesehen sind.

33. Biopsievorrichtung nach Anspruch 1 und 14, **dadurch gekennzeichnet, dass** die obere Außenkontur des Biopsienadelträgers (37) der Gehäuseinnenkontur entspricht.

34. Biopsievorrichtung nach Anspruch 1 und 14 , **dadurch gekennzeichnet, dass** auf dem rechten Ende (proximalen Ende) der Biopsienadel (2) ein Kunststoffteil (47) mit Rändelscheibe (80) kraftschlüssig aufgesetzt ist.

35. Biopsievorrichtung nach Anspruch 1 und 14, **dadurch gekennzeichnet, dass** das Kunststoffteil (47) einen Vielkant (50) aufweist, der mit dem Biopsienadelträger (37) zusammenwirkt und bei Verdrehen mittels der Rändelscheibe (80) die Biopsienadel (2) und damit der Probeentnahmeraums (71) in der gewählten Position im Biopsienadelträger (37) verrastet ist.

36. Biopsievorrichtung nach Anspruch 28, **dadurch gekennzeichnet, dass** durch eine mit dem Basisblock (8) verbundene Abdeckung (46) der Raum für die Getriebemotoren vom übrigen Raum nach oben getrennt wird.

37. Biopsievorrichtung nach Anspruch 1 und 28, **dadurch gekennzeichnet, dass** der Batterie-/Akkuraum nach oben durch eine Trennplatte (144) vom übrigen Raum getrennt ist.

38. Biopsievorrichtung nach Anspruch 17, **dadurch gekennzeichnet, dass** ein um eine Achse (35) unter Federdruck verstellbarer, doppelarmiger Hebel (33) verwendet wird, auf dessen einen Arm (100) eine Druckfeder (34) wirkt, und dessen anderer Arm (99) in eine Ausnehmung (82) des Spannschlittens (28) eingreift.

39. Biopsievorrichtung nach Anspruch 1, **dadurch gekennzeichnet, dass** in die Platine die Funktionsanzeige und Bedienschalter für die Elektronik integriert sind. (Fig. 7)

40. Biopsievorrichtung nach Anspruch 1, **dadurch gekennzeichnet, dass** das Verbindungselement (4) über ein drehbar im Kunststoffteil (47) gelagertes Kunststoffteil (112) mit der Biopsienadel (2) verbunden ist.

41. Biopsievorrichtung nach Anspruch 40, **dadurch gekennzeichnet, dass** das Kunststoffteil (112) gegenüber dem Kunststoffteil (47) mittels eines O-Rings abgedichtet ist.

42. Biopsievorrichtung nach Anspruch 14, **dadurch gekennzeichnet, dass** das herausnehmbare Element (20) eine steril verpackte Einheit ist.

43. Biopsievorrichtung nach Anspruch 14, **dadurch gekennzeichnet, dass** der Biopsienadelträger (37) und die Vakuum-/Druckerzeugungsvorrichtung (5), von Laschen (108), (118) einer Einlegehilfe umfasst werden und ein an der Einlegehilfe angebrachter Steg (109) den Biopsienadelträger (37) in der Längsachse ausrichtet und die Vakuum-/Druckerzeugungsvorrichtung durch ein in diese eingreifenden Stift (110) in der Längsachse ausgerichtet wird.

44. Biopsievorrichtung nach Anspruch 43, **dadurch gekennzeichnet, dass** die Einlegehilfe an der Oberseite zwei Haltestücke (117) aufweist.

45. Biopsievorrichtung nach Anspruch 1, **dadurch gekennzeichnet, dass** die Schneidhülse (3) beim Herausschneiden der Gewebeprobe über das distale Ende des Probeentnahmeraums um ca. 2 mm hinaus in Richtung Nadelspitze gefahren wird.

46. Biopsievorrichtung nach Anspruch 1, **dadurch gekennzeichnet, dass** bei Verwendung einer Koaxialkanüle zum Positionieren am proximalen Ende des Koaxialkanülenrohres eine Dichtung vorgesehen ist, die bei eingeführter Nadel einen Abbau des Vakuums verhindert.

47. Biopsievorrichtung nach Anspruch 45, **dadurch gekennzeichnet, dass** zwischen der proximalen Aufsetzfläche der Koaxialkanüle und der distalen Stirnfläche des Führungsrings ein oder mehrere Distanzstücke einsetzbar sind.

## Claims

1. A biopsy device for collecting tissue samples, comprising a hand-held piece into which a hollow biopsy needle is inserted, wherein part of the portion of the biopsy needle, including its sampling chamber, which protrudes from the hand-held piece is introduced into the tissue to be examined and the tissue is drawn into the sampling chamber by means of a vacuum and subsequently severed by means of a sample severing device and then collected, **characterized in that** the tensioning carriage is moved into the tensioned position by means of an electric motor and against the action of a spring, that the needle unit is mounted on the tensioning carriage supported within the hand-held piece, and that the sampling chamber is shot discharged into the tissue after unlatching of the pretensioned tensioning carriage, that the vacuum/pressure generating means (5) as well as additional control and supply means are integrated in the housing of the hand-held piece (1), and that the connection element (4) connecting the biopsy needle (3) to the vacuum/pressure generating unit (5) is provided directly on the housing, that said vacuum/pressure generating means (5) consists of a controllable piston/cylinder unit (69) including a ventilation aperture (67) to allow a positive pressure to be generated in the vacuum/pressure generating means for ejecting the sample, that all drives are actuated electrically and that the drive for the tensioning carriage can also be used as a drive for the cutting sleeve, that the hollow biopsy needle is surrounded by an external, coaxially disposed cutting sleeve and that a board is provided on the face of the housing for actuating the electronic systems, into which board the tensioning carriage unlatching means is integrated.

2. The biopsy device according to claim 1, **characterized in that** the vacuum/pressure generating means (5) is a syringe/piston unit which includes a ventilation aperture (67) in its upper portion which will be opened in order to relieve the vacuum by retracting the syringe piston (54).

3. The biopsy device according to claims 1 and 2, **characterized in that** the piston (54) of the vacuum/pressure generating means (5) can be moved in both directions by means of a controllable spindle drive (53, 48).

4. The biopsy device according to claim 3, **characterized in that** a high-speed D.C. electric motor (58) having a planetary gear located downstream is used to drive the controllable spindle drive (53, 48).

5. The biopsy device according to claim 4, **characterized in that** the transmission from the planetary gear to the piston spindle (53) is effected via a one-stage toothed gearing, with the threaded spindle nut (48) mounted on the syringe cylinder bearing a toothed ring (55) on the outside.

6. The biopsy device according to claims 1 through 4, **characterized in that** the piston (54) for generating a vacuum in the system and in the sampling chamber will in a first step be moved away from the syringe bottom (52) to a position just short of the ventilation aperture (67).

7. The biopsy device according to claims 1 through 4, **characterized in that**, in order to ventilate the system, in a second step following said first step, the piston (54) will be pulled back past the ventilation aperture (67), and moved back again once the vacuum has been relieved so as to close the ventilation bore.

8. The biopsy device according to claims 1 through 4, **characterized in that**, in a third step following said second step, the piston (54) will be moved towards the syringe bottom (52) so as to generate positive pressure in the system and in the sampling chamber.

9. The biopsy device according to claim 1, **characterized in that** the electric geared motor (58) of the piston/cylinder unit (69) is controlled in such a manner by means of speed measurement that the piston (54), in a first step, will be drawn out of the cylinder to a position just short of the ventilation aperture (67), in a second step, said ventilation aperture (67) will be exposed and said piston (54), after the vacuum has been relieved, will close the ventilation aperture, and, in a third step, will be moved in the opposite direction towards the syringe bottom (51) for generating a positive pressure, in step with the controlling of the sample collection and the ejection of the sample.

10. The biopsy device according to claim 1, **characterized in that** the speed of the D.C. motors is measured by a photocell fixedly mounted on the motor housing and a transmitter mounted on the motor shaft.

11. The biopsy device according to claim 10, **characterized in that**, after comparison with a desired value previously stored in the electronics, the motor speed will be used as trigger for the controlling of the spindle drive.

12. The biopsy device according to claim 1, **characterized in that** the interior of the hollow biopsy needle (2) is connected to the interior of the vacuum/pressure generating means (5) via a connection element (4) such that in a negative pressure situation, no air can enter from outside, and in a positive pressure situation, no air can escape.

13. The biopsy device according to claim 12, **characterized in that** the connecting piece (4) is a flexible tube mounted in the immediate vicinity of the hand-held piece (1).

14. The biopsy device according to one or several of the preceding claims, **characterized in that** the biopsy needle support (37) with the round, hollow biopsy needle (2) and the cutting sleeve (3), which also has a round cross section and is coaxially disposed around the biopsy needle, as well as parts of the drive, the connection element (4) and the vacuum/pressure generating means (5) form a removable, replaceable element (20) which can be inserted into the hand-held piece (1).

15. The biopsy device according to claim 14, **characterized in that** the recesses (77) provided in the removable biopsy needle support (37) will facilitate insertion of the latter into the mounting links (40) of the tensioning carriage (28).

16. The biopsy device according to claim 1, **characterized in that** the tensioning carriage (28) is moved into a tensioned position by a spindle drive (73, 75) driven by a D.C. geared motor (21) having a single-stage gear (23, 74) provided downstream thereof.

17. The biopsy device according to claim 17, **characterized in that** the tensioning carriage (28) can be mechanically locked in its tensioned position.

18. The biopsy device according to one or several of claims 14 through 17, **characterized in that** a toothed roller (23) is mounted on the driven shaft of the planetary gear downstream from the D.C. geared motor, which toothed roller (23) will engage the gearwheel (74) of the spindle drive (73, 75) connected to the cutting sleeve (3)

19. The biopsy device according to claim 18, **characterized in that** the gearwheel (74) mounted on the spindle drive (73, 75) rests on a support (36) of the basic block (8) when the tensioning carriage (28) is displaced.

20. The biopsy device according to claims 1 and 14, **characterized in that** the biopsy needle (2) including the coaxially disposed cutting sleeve (3) and additional elements mounted thereon is held inside the biopsy needle support (37) at two bearing points (75, 49) in such a manner that the biopsy needle (2) and/or the cutting sleeve (3) can be rotated individually.

21. The biopsy device according to claims 1 and 14, **characterized in that** the piston/cylinder unit (69) is designed in such a way that a vacuum of the order of 200 hph will be generated in the sampling chamber (71) of the system consisting mainly of the cavity piston/cylinder unit (69), the hollow biopsy needle (2) and the hollow connection piece (4).

22. The biopsy device according to claims 1 and 14, **characterized in that** the cross section of the biopsy needle in front of the sampling chamber includes a constriction (79) which covers the opening of the cross section of the sampling chamber from above.

23. The biopsy device according to claim 22, **characterized in that** said constriction comprises 60 % to 75 % of the cross section and that said constriction projects into the cross section from above.

24. The biopsy device according to claim 23, **characterized in that** the constriction is a plug being approx. 10 mm in length.

25. The biopsy device according to one of claims 1 to 14, **characterized in that** the constriction is designed in the form of a lip or nose which projects into the cross section.

26. The biopsy device according to claims 1 and 14, **characterized in that** only about 25% of the cross section of the sampling chamber (71) is open towards the top.

27. The biopsy device according to claim 20, **characterized in that** the threaded spindle nut (75) of the threaded spindle sleeve (73) is press-fitted into the biopsy needle support (37) and forms one of the two bearing sites.

28. The biopsy device according to claim 1, **characterized in that** a basic block (8) is provided at the center of the inner housing which block serves both for the fixation and as prop, support and clamping of the individual components such as tensioning carriage (28), biopsy needle support (37), vacuum/pressure generating means (3) and drive means (105, 106).

29. The biopsy device according to claim 1, **characterized in that** a microswitch (18) is integrated within the housing end cover (7) serving as support, on the side of the housing, for the vacuum/pressure generating means (5), with the actuation of said microswitch (18) releasing the energy supply.

30. The biopsy device according to claim 29, **characterized in that** the actuating pin (19) of the microswitch (18) is actuated by pressing down the vacuum/pressure generating means (5) by means of the housing cover (10).

31. The biopsy device according to claims 1 and 14, **characterized in that** means are provided on the biopsy needle support (37) which, when the tensioning carriage is tensioned and the biopsy needle support is inserted, will prevent closing of the housing cover (10), or opening of the housing cover if the housing is already closed.

32. The biopsy device according to claim 1, **characterized in that** surfaces are provided on the housing for attaching the hand-held piece to a positioning means.

33. The biopsy device according to claims 1 and 14, **characterized in that** the upper external contour of the biopsy needle support (37) corresponds to the internal contour of the housing.

34. The biopsy device according to claims 1 and 14, **characterized in that** a plastic part (47) including a knurled wheel has been clamped on the right end (i.e. the proximal end) of the biopsy needle (2).

35. The biopsy device according to claims 1 and 14, **characterized in that** the plastic part (47) includes a polyhedral element (50) which cooperates with the biopsy needle support (37) which, when twisted by means of the knurled wheel (80), will cause the biopsy needle (2) and thus the sampling chamber (71) to be latched in the chosen position in the biopsy needle support (37).

36. The biopsy device according to claim 28, **characterized in that**, through a cover (46) connected to the basic block (8), the space for the geared motors is separated from the rest of the space towards the top.

37. The biopsy device according to claims 1 and 28, **characterized in that** the space for the battery/storage battery is separated from the rest of the space towards the top by a separating plate (144).

38. The biopsy device according to claim 17, **characterized in that** a double-armed lever (33), which can be spring-biased to pivot about an axis (35) is used, with a pressure spring (34) acting on its one arm (100), and with its other arm (99) engaging a recess (82) of the tensioning carriage (28).

39. The biopsy device according to claim 1, **characterized in that** the function display and the operating switches for the electronics are integrated within the board (Fig. 7).

40. The biopsy device according to claim 1, **characterized in that** the connection element (4) is connected to the biopsy needle (2) via a plastic part (112) rotatably mounted within the plastic part (47).

41. The biopsy device according to claim 40, **characterized in that** the plastic part (112) is sealed from the plastic part (47) by means of an O ring.

42. The biopsy device according to claim 14, **characterized in that** the removable element (20) is a sterile packaged unit.

43. The biopsy device according to claim 14, **characterized in that** the biopsy needle support (37) and the vacuum/pressure generating means (5) are encompassed by the mounting links (108, 118) of an insertion aid, and a web (109) mounted on the insertion aid will orient the biopsy needle support (37) along the longitudinal axis, and the vacuum/pressure generating means will be oriented along its longitudinal axis by a pin (110) engaging the latter.

44. The biopsy device according to claim 43, **characterized in that** the insertion aid includes two grips (117) at its top.

45. The biopsy device according to claim 1, **characterized in that**, when cutting out the tissue sample, the cutting sleeve (3) will be moved beyond the distal end of the sample chamber by approx. 2 mm in the direction of the needle tip.

46. The biopsy device according to claim 1, **characterized in that**, when using a coaxial cannula for positioning, a sealing element is provided at the proximal end of the coaxial cannula tube, said sealing element preventing the vacuum from being relieved after the needle has been inserted.

47. The biopsy device according to claim 45, **characterized in that** one or several spacer(s) can be inserted between the proximal contact surface of the coaxial cannula and the distal face of the guide ring.

## Revendications

1. Dispositif pour biopsie permettant le prélèvement d'échantillons de tissu, constitué d'une pièce à main dans laquelle une aiguille de biopsie creuse est insérée, une partie de la partie, dépassant de la pièce à main, de l'aiguille de biopsie avec sa chambre de prélèvement d'échantillons étant introduite dans le tissu à analyser et le tissu étant aspiré au moyen du vide dans la chambre de prélèvement d'échantillons et ensuite coupé au moyen d'un dispositif de séparation d'échantillons puis prélevé, **caractérisé en ce que** le chariot de tension est amené en position de tension contre l'action d'un ressort au moyen d'une force motrice électrique, **en ce que** l'ensemble aiguille est disposé sur le chariot de tension positionné dans la pièce à main et la chambre de prélèvement d'échantillons est enfermée dans le tissu après déverrouillage du chariot de tension préalablement tendu, le dispositif de production de vide/de pression (5) ainsi que d'autres dispositifs de commande et d'alimentation sont intégrés dans le boîtier de la pièce à main (1) et l'élément de liaison (4) entre l'aiguille de biopsie (3) et l'élément de production de vide/de pression (5) est disposé directement sur le boîtier, le dispositif de production de vide/de pression (5) est constitué d'un ensemble piston-cylindre (69) pilotable qui présente une ouverture d'aération (67), de manière à pouvoir produire, dans le dispositif de production de vide/de pression, une surpression pour éjecter l'échantillon, **en ce que** tous les entraînements sont à commande électrique et **en ce que** l'entraînement du chariot de tension est aussi utilisé comme entraînement pour le morcellateur, **en ce que** l'aiguille de biopsie creuse est entourée d'un morcellateur coaxial situé à l'extérieur et **en ce que**, sur la face avant du boîtier, il y a un circuit imprimé destiné à l'actionnement de l'électronique dans lequel le déverrouilleur du chariot de tension est intégré.

2. Dispositif pour biopsie selon la revendication 1, **caractérisé en ce que** le dispositif de production de vide / de pression (5) est un ensemble seringue/piston qui présente, dans la partie supérieure, une ouverture d'aération (67) que l'on ouvre en faisant reculer le piston de la seringue (54) pour supprimer le vide.

3. Dispositif pour biopsie selon la revendication 1 et 2, **caractérisé en ce que** le piston (54) du dispositif de production de vide / de pression (5) peut se déplacer dans les deux sens au moyen d'un entraînement de broche pilotable (53, 48).

4. Dispositif pour biopsie selon la revendication 3, **caractérisé en ce que**, comme entraînement pour l'entraînement de broche pilotable (53, 48), on utilise un moteur électrique à courant continu à grande vitesse (58) comportant un engrenage planétaire en aval.

5. Dispositif pour biopsie selon la revendication 4, **caractérisé en ce que** la transmission depuis l'engrenage planétaire à la broche de piston (53) est assurée par un engrenage monoétagé, l'écrou de broche filetée (18), posé sur le cylindre de la seringue et situé à l'extérieur, porte une couronne dentée 55.

6. Dispositif pour biopsie selon la revendication 1 - 4, **caractérisé en ce que**, le piston (54) s'éloigne, dans une première étape, du fond de la seringue (52) jusqu'à peu avant l'ouverture d'aération (67) pour produire un vide dans le système et dans la chambre de prélèvement d'échantillons.

7. Dispositif pour biopsie selon la revendication 1 - 4, **caractérisé en ce que**, pour aérer le système, le piston (54), dans une deuxième étape suivant la première, est retiré au-delà de l'ouverture d'aération (67) et après avoir supprimé le vide, à nouveau reculé pour fermer le trou d'aération.

8. Dispositif pour biopsie selon la revendication 1 - 4, **caractérisé en ce qu'**on déplace le piston (54), dans une troisième étape suivant la deuxième, en direction du fond de la seringue (52) pour produire une surpression dans le système et dans la chambre de prélèvement d'échantillons.

9. Dispositif pour biopsie selon la revendication 1, **caractérisé en ce que** le moto-réducteur électrique (58) de l'ensemble piston/cylindre (69) est piloté, via une mesure de la vitesse, de manière à retirer le piston (54), dans une première étape, du cylindre jusqu'à peu avant l'ouverture d'aération (67), à libérer, dans une deuxième étape, le trou d'aération (67) est libéré et le piston, après suppression du vide, à refermer le trou d'aération et, dans une troisième étape, pour produire la surpression, à le déplacer dans le sens inverse vers le fond de la seringue (51) en accord avec la commande du prélèvement d'échantillons et l'éjection de l'échantillon.

10. Dispositif pour biopsie selon la revendication 1, **caractérisé en ce que** le nombre de rotation des moteurs à courant continu est mesuré par une cellule photoélectrique disposée sur le carter du moteur et un transmetteur disposé sur l'arbre du moteur.

11. Dispositif pour biopsie selon la revendication 10, **caractérisé en ce que** le nombre de rotations du moteur, comparé à une valeur de consigne enregistrée au préalable dans l'électronique, est utilisé comme déclencheur pour la commande de l'entraînement de broche.

12. Dispositif pour biopsie selon la revendication 1, **caractérisé en ce que** l'intérieur de l'aiguille de biopsie creuse (2) est relié à l'intérieur du dispositif de vide/de pression (5) via un élément de liaison (4) de manière à empêcher l'entrée d'air de l'extérieur en cas de dépression ou la sortie d'air en cas de surpression.

13. Dispositif pour biopsie selon la revendication 12, **caractérisé en ce que** l'élément de liaison (4) est un tuyau flexible disposé à proximité immédiate de la pièce à main (1).

14. Dispositif pour biopsie selon l'une quelconque ou plusieurs des revendications précédentes, **caractérisé en ce que** le porte-aiguille de biopsie (37), avec l'aiguille de biopsie circulaire creuse (2), et le morcellateur (3), de section également circulaire, entourant coaxialement l'aiguille de biopsie, ainsi que certaines pièces de l'entraînement, l'élément de liaison (4) et le dispositif de production de vide/de pression (5) forment un élément amovible interchangeable (20) qui peut être introduit dans la pièce à main (1).

15. Dispositif pour biopsie selon la revendication 14, **caractérisé en ce que** le porte-aiguille de biopsie (37) amovible peut être placé dans les colliers (40) du chariot de tension (28) au moyen des évidements (77).

16. Dispositif pour biopsie selon la revendication 1, **caractérisé en ce que** le chariot de tension (28) est amené en position de tension par l'intermédiaire d'un entraînement de broche (73, 75) entraîné au moyen d'un moto-réducteur à courant continu (21) comportant un engrenage (23, 74) monoétagé placé en aval.

17. Dispositif pour biopsie selon la revendication 17, **caractérisé en ce que** le chariot de tension (28) peut être verrouillé mécaniquement en position de tension.

18. Dispositif pour biopsie selon l'une quelconque ou plusieurs des revendications 14-17, **caractérisé en ce que**, sur l'arbre de sortie de l'engrenage planétaire placé en aval du moto-réducteur à courant continu, se trouve un rouleau denté (23) dans lequel la roue dentée (74) de l'entraînement de broche (73, 75), relié au morcellateur (3), entre en prise.

19. Dispositif pour biopsie selon la revendication 18, **caractérisé en ce que** la roue dentée (74) posée sur l'entraînement de broche (73, (75), s'appuie sur une attache (36) du bloc de base (8) lors du déplacement du chariot de tension (28).

20. Dispositif pour biopsie selon la revendication 1 et 14, **caractérisé en ce que** l'aiguille de biopsie (2), avec le morcellateur coaxial (3) et d'autres éléments disposés contre, sont maintenus dans le porte-aiguille de biopsie (37) contre deux points d'appui (75, 49) de manière ce que l'aiguille de biopsie (2) et/ou le morcellateur (3) puissent tourner chacun pour soi.

21. Dispositif pour biopsie selon la revendication 1 et 14, **caractérisé en ce que** l'ensemble piston/cylindre (69) est conçu de manière à créer un vide de l'ordre de 200 hph dans le système constitué surtout de l'ensemble piston/cylindre creux (69), de l'aiguille de biopsie creuse (2) et de l'élément de liaison creux.

22. Dispositif pour biopsie selon la revendication 1 et 14, **caractérisé en ce que** la section de l'aiguille de biopsie présente, avant la chambre de prélèvement d'échantillons, un rétrécissement (79) qui recouvre l'ouverture de la section de la zone de prélèvement d'échantillons depuis le haut.

23. Dispositif pour biopsie selon la revendication 22, **caractérisé en ce que** le rétrécissement englobe 60 à 75 % de la section et **en ce que** le rétrécissement empiète dans la section par le haut.

24. Dispositif pour biopsie selon la revendication 23, **caractérisé en ce que** le rétrécissement est un bouchon d'environ 10 mm de long.

25. Dispositif pour biopsie selon la revendication 1 et 14, **caractérisé en ce que** le rétrécissement est en forme de lèvre ou de nez empiétant dans la section.

26. Dispositif pour biopsie selon la revendication 1 et 14, **caractérisé en ce que** seulement 25% environ de la section de la chambre de prélèvement d'échantillons (71) sont ouverts vers le haut.

27. Dispositif pour biopsie selon la revendication 20, **caractérisé en ce que** l'écrou de broche filetée (75) du manchon de broche filetée (73) est pressé dans le porte-aiguille de biopsie (37) et forme l'un des deux points d'appui.

28. Dispositif pour biopsie selon la revendication 1, **caractérisé en ce que**, au centre du boîtier intérieur, il y a un bloc de base (8) qui sert aussi bien à la fixation qu'au soutien, au positionnement et au maintien des différents composants comme le chariot de tension (28), le porte-aiguille de biopsie (37), le dispositif de production de vide/de pression (3) et les dispositifs d'entraînement (105, 106).

29. Dispositif pour biopsie selon la revendication 1, **caractérisé en ce que**, dans le couvercle terminal du boîtier (7), servant au positionnement, côté boîtier, du dispositif de production de vide/de pression (5), est intégré un microrupteur (18) qui active l'alimentation en énergie lorsqu'on l'actionne.

30. Dispositif pour biopsie selon la revendication 29, **caractérisé en ce qu'**on actionne la tige de commutation (19) du microrupteur (18) en enfonçant le dispositif de production de vide/de pression (5) au moyen du couvercle de boîtier (10).

31. Dispositif pour biopsie selon la revendication 1 et 14, **caractérisé en ce que**, sur le porte-aiguille de biopsie (37) sont prévus des moyens qui empêchent une fermeture du couvercle du boîtier (10) respectivement une ouverture du couvercle de boîtier, lorsque le boîtier est fermé, lorsque le chariot de tension est tendu et le porte-aiguille de biopsie est en place.

32. Dispositif pour biopsie selon la revendication 1, **caractérisé en ce que** des surfaces sont prévues sur le boîtier pour fixer la pièce à main contre un dispositif de positionnement.

33. Dispositif pour biopsie selon la revendication 1 et 14, **caractérisé en ce que** le contour extérieur supérieur du porte-aiguille de biopsie (37) correspond au contour intérieur du boîtier.

34. Dispositif pour biopsie selon la revendication 1 et 14, **caractérisé en ce qu'**une pièce plastique (47) comportant une molette (80) est posée par adhérence sur l'extrémité droite (extrémité proximale) de l'aiguille de biopsie (2).

35. Dispositif pour biopsie selon la revendication 1 et 14, **caractérisé en ce que** la pièce plastique (47) présente un carré (50) qui interagit avec le porte-aiguille de biopsie (37) et qui verrouille l'aiguille de biopsie (2) et ainsi la chambre de prélèvement d'échantillons (71) dans la position choisie dans le porte-aiguille de biopsie (37) lorsqu'on tourne au moyen de la molette (80).

36. Dispositif pour biopsie selon la revendication 28, **caractérisé en ce qu'**un recouvrement (46), relié au bloc de base (8) permet de séparer vers le haut l'espace destiné aux moto-réducteurs du reste de l'espace.

37. Dispositif pour biopsie selon la revendication 1 et 28, **caractérisé en ce que** le logement pour une pile/accu est séparé vers le haut du reste de l'espace par une plaque de séparation (144).

38. Dispositif pour biopsie selon la revendication 17, **caractérisé en ce qu'**on utilise un levier (33) à deux branches pouvant se déplacer autour d'un axe (35) par une force élastique, levier sur une branche (100) duquel agit un ressort de pression (34) et dont l'autre branche (99) entre en prise dans un évidement (82) du chariot de tension (28).

39. Dispositif pour biopsie selon la revendication 1, **caractérisé en ce que** l'afficheur des fonctions et le commutateur de l'électronique sont intégrés dans le circuit imprimé (Fig. 7).

40. Dispositif pour biopsie selon la revendication 1, **caractérisé en ce que** l'élément de liaison (4) est relié à l'aiguille de biopsie (2) par l'intermédiaire d'une pièce plastique (112) logée dans la pièce plastique (47) de manière à pouvoir pivoter.

41. Dispositif pour biopsie selon la revendication 40, **caractérisé en ce que** la pièce plastique (112) est étanche, par rapport à la pièce plastique (47) au moyen d'un joint torique.

42. Dispositif pour biopsie selon la revendication 14, **caractérisé en ce que** l'élément amovible (20) est un ensemble emballé de manière stérile.

43. Dispositif pour biopsie selon la revendication 14, **caractérisé en ce que** le porte-aiguille de biopsie (37) et le dispositif de production de vide/de pression (5) sont entourés de colliers (108), (118) d'un accessoire d'introduction et une entretoise (109) placée contre l'accessoire d'introduction oriente le porte-aiguille de biopsie (37) dans l'axe longitudinal et le dispositif de production de vide/de pression est orienté dans l'axe longitudinal par une tige (110) entrant en prise dans ce dispositif.

44. Dispositif pour biopsie selon la revendication 43 **caractérisé en ce que** l'accessoire d'introduction présente deux éléments de retenue (117) sur le côté supérieur.

45. Dispositif pour biopsie selon la revendication 1, **caractérisé en ce que** le morcellateur (3) se déplace d'environ 2 mm au-delà de l'extrémité distale de la chambre de prélèvement d'échantillons en direction de la pointe de l'aiguille lors de la découpe de l'échantillon de tissu.

46. Dispositif pour biopsie selon la revendication 1, **caractérisé en ce qu'**on prévoit un joint, qui empêche la suppression du vide lorsque l'aiguille a été introduite, lorsqu'on utilise une canule coaxiale pour le positionnement à l'extrémité proximale du tube de la canule coaxiale.

47. Dispositif pour biopsie selon la revendication 45, **caractérisé en ce qu'**un ou plusieurs écarteurs peuvent être placés entre l'assise proximale de la canule coaxiale et la face avant distale de la bague de guidage.
